(19) Europäisches Patentamt
European Patent Office
Office européen des brevets

(11) EP 3 536 782 A1

(12) **EUROPEAN PATENT APPLICATION**
published in accordance with Art. 153(4) EPC

(43) Date of publication:
**11.09.2019 Bulletin 2019/37**

(21) Application number: **17866696.2**

(22) Date of filing: **01.11.2017**

(51) Int Cl.:
***C12N 15/09*** *(2006.01)* ***C12N 9/02*** *(2006.01)*
***C12P 21/02*** *(2006.01)*

(86) International application number:
**PCT/JP2017/039473**

(87) International publication number:
**WO 2018/084165 (11.05.2018 Gazette 2018/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **01.11.2016 JP 2016214073**

(71) Applicant: **Kaneka Corporation**
**Osaka-shi, Osaka 530-8288 (JP)**

(72) Inventors:
- **MATSUI, Misato**
  **Hyogo 676-8688 (JP)**
- **ITO, Noriyuki**
  **Hyogo 676-8688 (JP)**
- **YASOHARA, Yoshihiko**
  **Hyogo 676-8688 (JP)**

(74) Representative: **Hoffmann Eitle**
**Patent- und Rechtsanwälte PartmbB**
**Arabellastraße 30**
**81925 München (DE)**

# (54) MODIFIED ENZYME AND USE THEREOF

(57) The present invention provides, by modifying a wild-type glutathione synthetase, a modified glutathione synthetase which has a more stably retained activity, particularly a higher thermal stability, and/or a transformant capable of producing the modified glutathione synthetase.

A polypeptide in accordance with the present invention is a polypeptide which (a) is capable of carrying out a reaction of binding glycine to γ-glutamyl dipeptide and (b) has a higher thermal stability and/or a higher storage stability as compared with glutathione synthetase consisting of an amino acid sequence of SEQ ID NO: 1 shown in the Sequence Listing.

EP 3 536 782 A1

**Description**

Technical Field

The present invention relates to a modified glutathione synthetase, a gene encoding the modified glutathione synthetase, and use of the modified glutathione synthetase and the gene.

Background Art

Glutathione is a peptide consisting of the three amino acids L-cysteine, L-glutamic acid, and glycine, and is present not only in a human body but also in many organisms such as animals other than humans, plants, and microorganisms. Glutathione has functions such as active oxygen elimination, detoxification, and amino acid metabolism, and is thus an important compound for organisms.

In an organism, glutathione is present either in the form of reduced glutathione (hereinafter also referred to as "GSH"), in which a thiol group of an L-cysteine residue is reduced so as to have a SH form, or in the form of oxidized glutathione (hereinafter also referred to as "GSSG"), in which thiol groups of an L-cysteine residue are oxidized so that a disulfide bond is formed between two glutathione molecules.

As for a method of producing glutathione, for example, there has been known an enzyme method in which bacterial cells of *Escherichia coli* or *saccharomyces cerevisiae*, in which γ-glutamyl cysteine synthetase or glutathione synthetase has been produced by recombination, are used as an enzyme source in the presence of L-glutamic acid, L-cysteine, glycine, and a surfactant and/or an organic solvent (Patent Literatures 1 and 2). Further, the Applicant recently published a method for producing oxidized glutathione including the steps of producing oxidized γ-glutamyl cysteine with use of L-glutamic acid and L-cystine and subsequently producing oxidized glutathione with use of the oxidized γ-glutamyl cysteine and glycine (Patent Literature 3).

As enzymes associated with glutathione synthesis, γ-glutamyl cysteine synthetase (hereinafter also referred to as "GSHI"), which produces γ-glutamyl cysteine by binding L-glutamic acid and L-cysteine, and glutathione synthetase (hereinafter also referred to as "GSHII"), which produces reduced glutathione by binding γ-glutamyl cysteine and glycine, are known. Further, GSHI and GSHII are each known to be also capable of using L-cystine and oxidized γ-glutamyl cysteine as substrates, and in such a case, oxidized γ-glutamyl cysteine and oxidized glutathione are synthesized as a product from a GSHI enzymatic reaction and a product from a GSHII enzymatic reaction, respectively (Patent Literature 3).

Citation List

[Patent Literature]

[Patent Literature 1]
Japanese Patent Application Publication, *Tokukaishou,* No. 60-27396 (Publication Date: February 12, 1985)
[Patent Literature 2] Japanese Patent Application Publication, *Tokukaishou*, No. 60-27397 (Publication Date: February 12, 1985)
[Patent Literature 3] International Publication No. 2016/002884 (publication date: January 7, 2016)


[Non-patent Literature]

[Non-Patent Literature 1] Appl. Microbiol. Biotechnol., 66, 233 (2004)
[Non-Patent Literature 2] Appl. Environ. Microbiol., 44, 1444 (1982)


Summary of Invention

Technical Problem

In industrial-scale production of glutathione with use of glutathione synthetase, the glutathione synthetase needs to have storage stability and thermal stability at a reaction temperature. Further, in conducting a reaction using recombinant bacterial cells expressing glutathione synthetase, if the glutathione synthetase has a high stability, it is possible to suppress background reactions of host-derived enzymes other than the glutathione synthetase by heating the recombinant bacterial cells so as to deactivate or decrease activities of the host-derived enzymes.

It is therefore an object of the present invention to provide a modified glutathione synthetase which, as compared with wild-type glutathione synthetase, has a more stably retained activity, particularly a higher thermal stability, and/or a transformant capable of producing the modified glutathione synthetase.

Solution to Problem

The inventors of the present invention, through diligent study, successfully discovered a modified glutathione synthetase having an improved thermal stability as compared with wild-type glutathione synthetase, from among a library (hereinafter referred to as "mutant enzyme gene library") of modified glutathione synthetase genes produced by introducing a mutation to a wild-type glutathione synthetase gene. Thus, the inventors of the present invention completed the present invention.

That is, an aspect of the present invention encompasses the following.

[1] A polypeptide exhibiting properties (a) and (b) below:

(a) being capable of carrying out a reaction of binding glycine to γ-glutamyl dipeptide; and
(b) having a higher thermal stability and/or a higher storage stability as compared with glutathione synthetase consisting of an amino acid sequence of SEQ ID NO: 1 shown in the Sequence Listing.

[2] A polypeptide exhibiting properties (c) and (d) below:

(c) being capable of producing reduced glutathione (GSH) and/or oxidized glutathione (GSSG); and
(d) having a higher thermal stability and/or a higher storage stability as compared with glutathione synthetase consisting of an amino acid sequence of SEQ ID NO: 1 shown in the Sequence Listing.

[3] A polypeptide according to [1], wherein the polypeptide is capable of: producing GSH and GSSG with use of γ-glutamyl cysteine and oxidized γ-glutamyl cysteine as substrates; producing GSH with use of γ-glutamyl cysteine as a substrate; or producing GSSG with use of oxidized γ-glutamyl cysteine as a substrate.
[4] A polypeptide defined in any one of (A) to (C) below:

(A) a polypeptide according to any one of [1] to [3], wherein the polypeptide consists of an amino acid sequence which is obtained by substitution of one or more amino acids in the amino acid sequence of SEQ ID NO: 1 shown in the Sequence Listing, the one or more amino acids being selected from the group consisting of amino acids at respective positions 13, 17, 20, 23, 39, 70, 78, 101, 113, 125, 126, 136, 138, 149, 152, 154, 155, 197, 200, 215, 226, 227, 230, 239, 241, 246, 249, 254, 260, 262, 263, 270, 278, 299, 305, 307, and 310;
(B) a polypeptide according to any one of [1] to [3], wherein the polypeptide consists of an amino acid sequence which is obtained by substitution of one or more amino acids in the amino acid sequence of SEQ ID NO: 1 shown in the Sequence Listing, the one or more amino acids being selected from the group consisting of amino acids at respective positions 13, 17, 20, 23, 39, 70, 78, 101, 113, 125, 126, 136, 138, 149, 152, 154, 155, 197, 200, 215, 226, 227, 230, 239, 241, 246, 249, 254, 260, 262, 263, 270, 278, 299, 305, 307, and 310, and in which one or more amino acids at a position(s) other than said positions are substituted, added, inserted, or deleted; and
(C) a polypeptide according to any one of [1] to [3], wherein the polypeptide consists of an amino acid sequence which is obtained by substitution of one or more amino acids in the amino acid sequence of SEQ ID NO: 1 shown in the Sequence Listing, the one or more amino acids being selected from the group consisting of amino acids at respective positions 13, 17, 20, 23, 39, 70, 78, 101, 113, 125, 126, 136, 138, 149, 152, 154, 155, 197, 200, 215, 226, 227, 230, 239, 241, 246, 249, 254, 260, 262, 263, 270, 278, 299, 305, 307, and 310, and which has a sequence identity of 80% or more with respect to the amino acid sequence of SEQ ID NO: 1 shown in the Sequence Listing except for said positions.

[5] A polypeptide defined in any one of (D) to (F) below:

(D) a polypeptide according to any one of [1] to [3], wherein the polypeptide consists of an amino acid sequence which is obtained by substitution of one or more amino acids in the amino acid sequence of SEQ ID NO: 1 shown in the Sequence Listing, the substitution of the one or more amino acids being selected from the group consisting of: substitution of an amino acid at position 13 to serine; substitution of an amino acid at position 17 to glutamic acid; substitution of an amino acid at position 20 to threonine; substitution of an amino acid at position 23 to leucine; substitution of an amino acid at position 39 to threonine; substitution of an amino acid at position 70 to serine; substitution of an amino acid at position 78 to leucine; substitution of an amino acid at position 101 to asparagine, glutamine, serine, or threonine; substitution of an amino acid at position 113 to histidine; substitution of an amino acid at position 125 to valine; substitution of an amino acid at position 126 to asparagine; substitution of an amino acid at position 136 to threonine; substitution of an amino acid at position 138 to alanine; substitution of an amino acid at position 149 to glutamine; substitution of an amino acid at position 152 to glutamine; substitution of an amino acid at position 154 to asparagine; substitution of an amino acid at position 155 to leucine; substitution of an amino acid at position 197 to glutamine; substitution of an amino acid at position 200 to serine; substitution of an amino acid at position 215 to asparagine acid; substitution of an amino acid at position 226 to arginine; substitution of an amino acid at position 227 to serine; substitution of an amino acid at position 230 to proline; substitution of an amino acid at position 239 to serine; substitution of an amino acid at position 241 to histidine; substitution of an amino acid at position 246 to arginine; substitution of an amino acid at position 249 to glutamic acid; substitution of an amino acid at position 254 to asparagine acid; substitution of an amino acid at position 260 to alanine, cystein, glycine, glutamine, or threonine; substitution of an amino acid at position 262 to cysteine; substitution of an amino acid at position 263 to arginine; substitution of an amino acid at position 270 to isoleucine; substitution of an amino acid at a position 278 to glycine or alanine; substitution of an amino acid at position 299 to alanine; substitution of an amino acid at position 305 to glycine; substitution of an amino acid at position 307 to valine; and substitution of an amino acid at position 310 to threonine; and

(E) a polypeptide according to any one of [1] to [3], wherein the polypeptide consists of an amino acid sequence which is obtained by substitution of one or more amino acids in the amino acid sequence of SEQ ID NO: 1 shown in the Sequence Listing, the substitution of the one or more amino acids being selected from the group consisting of: substitution of an amino acid at position 13 to serine; substitution of an amino acid at position 17 to glutamic acid; substitution of an amino acid at position 20 to threonine; substitution of an amino acid at position 23 to leucine; substitution of an amino acid at position 39 to threonine; substitution of an amino acid at position 70 to serine; substitution of an amino acid at position 78 to leucine; substitution of an amino acid at position 101 to asparagine, glutamine, serine, or threonine; substitution of an amino acid at position 113 to histidine; substitution of an amino acid at position 125 to valine; substitution of an amino acid at position 126 to asparagine; substitution of an amino acid at position 136 to threonine; substitution of an amino acid at position 138 to alanine; substitution of an amino acid at position 149 to glutamine; substitution of an amino acid at position 152 to glutamine; substitution of an amino acid at position 154 to asparagine; substitution of an amino acid at position 155 to leucine; substitution of an amino acid at position 197 to glutamine; substitution of an amino acid at position 200 to serine; substitution of an amino acid at position 215 to asparagine acid; substitution of an amino acid at position 226 to arginine; substitution of an amino acid at position 227 to serine; substitution of an amino acid at position 230 to proline; substitution of an amino acid at position 239 to serine; substitution of an amino acid at position 241 to histidine; substitution of an amino acid at position 246 to arginine; substitution of an amino acid at position 249 to glutamic acid; substitution of an amino acid at position 254 to asparagine acid; substitution of an amino acid at position 260 to alanine, cystein, glycine, glutamine, or threonine; substitution of an amino acid at position 262 to cysteine; substitution of an amino acid at position 263 to arginine; substitution of an amino acid at position 270 to isoleucine; substitution of an amino acid at a position 278 to glycine or alanine; substitution of an amino acid at position 299 to alanine; substitution of an amino acid at position 305 to glycine; substitution of an amino acid at position 307 to valine; and substitution of an amino acid at position 310 to threonine, and in which one or more amino acids at a position(s) other than said positions are substituted, added, inserted, or deleted; and

(F) a polypeptide according to any one of [1] to [3], wherein the polypeptide consists of an amino acid sequence which is obtained by substitution of one or more amino acids in the amino acid sequence of SEQ ID NO: 1 shown in the Sequence Listing, the substitution of the one or more amino acids being selected from the group consisting of: substitution of an amino acid at position 13 to serine; substitution of an amino acid at position 17 to glutamic acid; substitution of an amino acid at position 20 to threonine; substitution of an amino acid at position 23 to leucine; substitution of an amino acid at position 39 to threonine; substitution of an amino acid at position 70 to serine; substitution of an amino acid at position 78 to leucine; substitution of an amino acid at position 101 to asparagine, glutamine, serine, or threonine; substitution of an amino acid at position 113 to histidine; substitution of an amino acid at position 125 to valine; substitution of an amino acid at position 126 to asparagine; substitution of an amino acid at position 136 to threonine; substitution of an amino acid at position 138 to alanine; substitution of an amino acid at position 149 to glutamine; substitution of an amino acid at position 152 to glutamine; substitution of an amino acid at position 154 to asparagine; substitution of an amino acid at position 155 to leucine; substitution of an amino acid at position 197 to glutamine; substitution of an amino acid at position 200 to serine; substitution of an amino acid at position 215 to asparagine acid; substitution of an amino acid at position 226 to arginine; substitution of an amino acid at position 227 to serine; substitution of an amino acid at position 230 to proline; substitution of an amino acid at position 239 to serine; substitution of an amino acid at position 241 to histidine; substitution of an amino acid at position 246 to arginine; substitution of an amino acid at position 249 to glutamic acid; substitution of an amino acid at position 254 to asparagine acid; substitution of an amino acid at position 260 to alanine, cystein, glycine, glutamine, or threonine; substitution of an amino acid at position 262 to cysteine; substitution of an amino acid at position 263 to arginine; substitution of an amino acid at position 270 to isoleucine; substitution of an amino acid at a position 278 to glycine or alanine; substitution of an amino acid at position 299 to alanine; substitution of an amino acid at position 305 to glycine; substitution of an amino acid at position 307 to valine; and substitution of an amino acid at position 310 to threonine, and which has a sequence identity of 80% or more with respect to the amino acid sequence of SEQ ID NO: 1 shown in the Sequence Listing except for said positions.

[6] A polypeptide defined in any one of (G) to (I) below:

(G) a polypeptide according to any one of [1] to [3], wherein the polypeptide consists of an amino acid sequence which is obtained by amino acid substitution in the amino acid sequence of SEQ ID NO: 1 shown in the Sequence Listing, the amino acid substitution being selected from the group consisting of:

(1) substitution of an amino acid at position 13 to serine;
(2) substitution of an amino acid at position 17 to glutamic acid, an amino acid at position 113 to histidine, and an amino acid at position 230 to proline;
(3) substitution of an amino acid at position 20 to threonine and an amino acid at position 215 to asparagine acid;
(4) substitution of an amino acid at position 20 to threonine and an amino acid at position 241 to histidine;
(5) substitution of an amino acid at position 23 to leucine and an amino acid at position 126 to asparagine;
(6) substitution of an amino acid at position 39 to threonine and an amino acid at position 260 to alanine;
(7) substitution of an amino acid at position 70 to serine and an amino acid at position 260 to alanine;
(8) substitution of an amino acid at position 78 to leucine and an amino acid at position 278 to alanine;
(9) substitution of an amino acid at position 101 to asparagine;
(10) substitution of an amino acid at position 101 to glutamine;
(11) substitution of an amino acid at position 101 to serine;
(12) substitution of an amino acid at position 101 to serine and an amino acid at position 260 to alanine;
(13) substitution of an amino acid at position 101 to threonine;
(14) substitution of an amino acid at position 125 to valine and an amino acid at position 249 to glutamic acid;
(15) substitution of an amino acid at position 125 to valine and an amino acid at position 152 to glutamine;
(16) substitution of an amino acid at position 136 to threonine;
(17) substitution of an amino acid at position 138 to alanine, an amino acid at position 149 to glutamine, an amino acid at position 241 to histidine, and an amino acid at position 263 to glutamine;
(18) substitution of an amino acid at position 154 to asparagine and an amino acid at position 246 to arginine;
(19) substitution of an amino acid at position 155 to leucine and an amino acid at position 239 to serine;
(20) substitution of an amino acid at position 197 to glutamine;
(21) substitution of an amino acid at position 200 to serine and an amino acid at position 260 to alanine;
(22) substitution of an amino acid at position 226 to arginine and an amino acid at position 260 to alanine;
(23) substitution of an amino acid at position 227 to serine and an amino acid at position 260 to alanine;
(24) substitution of an amino acid at position 254 to asparagine acid and an amino acid at position 260 to alanine;
(25) substitution of an amino acid at position 260 to alanine;
(26) substitution of an amino acid at position 260 to alanine, an amino acid at position 278 to glycine, and an amino acid at position 307 to valine;
(27) substitution of an amino acid at position 260 to alanine and an amino acid at position 299 to alanine;
(28) substitution of an amino acid at position 260 to alanine and an amino acid at position 305 to glycine;
(29) substitution of an amino acid at position 260 to alanine and an amino acid at position 310 to threonine;
(30) substitution of an amino acid at position 260 to cysteine;
(31) substitution of an amino acid at position 260 to glycine;
(32) substitution of an amino acid at position 260 to glutamine;
(33) substitution of an amino acid at position 260 to threonine;
(34) substitution of an amino acid at position 262 to cysteine; and
(35) substitution of an amino acid at position 270 to isoleucine;

(H) a polypeptide according to any one of [1] to [3], wherein the polypeptide consists of an amino acid sequence which is obtained by amino acid substitution in the amino acid sequence of SEQ ID NO: 1 shown in the Sequence Listing, the amino acid substitution being selected from the group consisting of:

(1) substitution of an amino acid at position 13 to serine;
(2) substitution of an amino acid at position 17 to glutamic acid, an amino acid at position 113 to histidine, and an amino acid at position 230 to proline;
(3) substitution of an amino acid at position 20 to threonine and an amino acid at position 215 to asparagine acid;
(4) substitution of an amino acid at position 20 to threonine and an amino acid at position 241 to histidine;
(5) substitution of an amino acid at position 23 to leucine and an amino acid at position 126 to asparagine;
(6) substitution of an amino acid at position 39 to threonine and an amino acid at position 260 to alanine;
(7) substitution of an amino acid at position 70 to serine and an amino acid at position 260 to alanine;
(8) substitution of an amino acid at position 78 to leucine and an amino acid at position 278 to alanine;
(9) substitution of an amino acid at position 101 to asparagine;
(10) substitution of an amino acid at position 101 to glutamine;
(11) substitution of an amino acid at position 101 to serine;
(12) substitution of an amino acid at position 101 to serine and an amino acid at position 260 to alanine;
(13) substitution of an amino acid at position 101 to threonine;
(14) substitution of an amino acid at position 125 to valine and an amino acid at position 249 to glutamic acid;
(15) substitution of an amino acid at position 125 to valine and an amino acid at position 152 to glutamine;
(16) substitution of an amino acid at position 136 to threonine;
(17) substitution of an amino acid at position 138 to alanine, an amino acid at position 149 to glutamine, an amino acid at position 241 to histidine, and an amino acid at position 263 to glutamine;
(18) substitution of an amino acid at position 154 to asparagine and an amino acid at position 246 to arginine;
(19) substitution of an amino acid at position 155 to leucine and an amino acid at position 239 to serine;
(20) substitution of an amino acid at position 197 to glutamine;
(21) substitution of an amino acid at position 200 to serine and an amino acid at position 260 to alanine;
(22) substitution of an amino acid at position 226 to arginine and an amino acid at position 260 to alanine;
(23) substitution of an amino acid at position 227 to serine and an amino acid at position 260 to alanine;
(24) substitution of an amino acid at position 254 to asparagine acid and an amino acid at position 260 to alanine;
(25) substitution of an amino acid at position 260 to alanine;
(26) substitution of an amino acid at position 260 to alanine, an amino acid at position 278 to glycine, and an amino acid at position 307 to valine;
(27) substitution of an amino acid at position 260 to alanine and an amino acid at position 299 to alanine;
(28) substitution of an amino acid at position 260 to alanine and an amino acid at position 305 to glycine;
(29) substitution of an amino acid at position 260 to alanine and an amino acid at position 310 to threonine;
(30) substitution of an amino acid at position 260 to cysteine;
(31) substitution of an amino acid at position 260 to glycine;
(32) substitution of an amino acid at position 260 to glutamine;
(33) substitution of an amino acid at position 260 to threonine;
(34) substitution of an amino acid at position 262 to cysteine; and
(35) substitution of an amino acid at position 270 to isoleucine, and in which one or more amino acids at a position(s) other than said positions are substituted, added, inserted, or deleted; and

(I) a polypeptide according to any one of [1] to [3], wherein the polypeptide consists of an amino acid sequence which is obtained by amino acid substitution in the amino acid sequence of SEQ ID NO: 1 shown in the Sequence Listing, the amino acid substitution being selected from the group consisting of:

(1) substitution of an amino acid at position 13 to serine;
(2) substitution of an amino acid at position 17 to glutamic acid, an amino acid at position 113 to histidine, and an amino acid at position 230 to proline;
(3) substitution of an amino acid at position 20 to threonine and an amino acid at position 215 to asparagine acid;
(4) substitution of an amino acid at position 20 to threonine and an amino acid at position 241 to histidine;
(5) substitution of an amino acid at position 23 to leucine and an amino acid at position 126 to asparagine;
(6) substitution of an amino acid at position 39 to threonine and an amino acid at position 260 to alanine;

(7) substitution of an amino acid at position 70 to serine and an amino acid at position 260 to alanine;
(8) substitution of an amino acid at position 78 to leucine and an amino acid at position 278 to alanine;
(9) substitution of an amino acid at position 101 to asparagine;
(10) substitution of an amino acid at position 101 to glutamine;
(11) substitution of an amino acid at position 101 to serine;
(12) substitution of an amino acid at position 101 to serine and an amino acid at position 260 to alanine;
(13) substitution of an amino acid at position 101 to threonine;
(14) substitution of an amino acid at position 125 to valine and an amino acid at position 249 to glutamic acid;
(15) substitution of an amino acid at position 125 to valine and an amino acid at position 152 to glutamine;
(16) substitution of an amino acid at position 136 to threonine;
(17) substitution of an amino acid at position 138 to alanine, an amino acid at position 149 to glutamine, an amino acid at position 241 to histidine, and an amino acid at position 263 to glutamine;
(18) substitution of an amino acid at position 154 to asparagine and an amino acid at position 246 to arginine;
(19) substitution of an amino acid at position 155 to leucine and an amino acid at position 239 to serine;
(20) substitution of an amino acid at position 197 to glutamine;
(21) substitution of an amino acid at position 200 to serine and an amino acid at position 260 to alanine;
(22) substitution of an amino acid at position 226 to arginine and an amino acid at position 260 to alanine;
(23) substitution of an amino acid at position 227 to serine and an amino acid at position 260 to alanine;
(24) substitution of an amino acid at position 254 to asparagine acid and an amino acid at position 260 to alanine;
(25) substitution of an amino acid at position 260 to alanine;
(26) substitution of an amino acid at position 260 to alanine, an amino acid at position 278 to glycine, and an amino acid at position 307 to valine;
(27) substitution of an amino acid at position 260 to alanine and an amino acid at position 299 to alanine;
(28) substitution of an amino acid at position 260 to alanine and an amino acid at position 305 to glycine;
(29) substitution of an amino acid at position 260 to alanine and an amino acid at position 310 to threonine;
(30) substitution of an amino acid at position 260 to cysteine;
(31) substitution of an amino acid at position 260 to glycine;
(32) substitution of an amino acid at position 260 to glutamine;
(33) substitution of an amino acid at position 260 to threonine;
(34) substitution of an amino acid at position 262 to cysteine; and
(35) substitution of an amino acid at position 270 to isoleucine, and which has a sequence identity of 80% or more with respect to the amino acid sequence of SEQ ID NO: 1 shown in the Sequence Listing except for said positions.

[7] A polynucleotide encoding a polypeptide according to any one of [1] to [6].
[8] A method for producing γ-Glu-X-Gly where X represents an amino acid, the method comprising causing a transformant expressing a polypeptide according to any one of [1] to [6] or a polynucleotide according to [7] and/or a treated material of the transformant to act on γ-glutamyl dipeptide.
[9] A method for producing GSSG, comprising causing a transformant expressing a polypeptide according to any one of [1] to [6] or a polynucleotide according to [7] and/or a treated material of the transformant to act on oxidized γ-glutamyl cysteine.
[10] A method for producing GSH, comprising causing a transformant expressing a polypeptide according to any one of [1] to [6] or a polynucleotide according to [7] and/or a treated material of the transformant to act on γ-glutamyl cysteine.
[11] The method according to [8], wherein a reaction is carried out in the coexistence of an ATP regenerating system.
[12] The method according to [11], wherein polyphosphate kinase is used as the ATP regenerating system.
[13] The method according to [9], wherein a reaction is carried out in the coexistence of an ATP regenerating system.
[14] The method according to [13], wherein polyphosphate kinase is used as the ATP regenerating system.
[15] The method according to [10], wherein a reaction is carried out in the coexistence of an ATP regenerating system.
[16] The method according to [15], wherein polyphosphate kinase is used as the ATP regenerating system.

Advantageous Effects of Invention

According to the aspect of the present invention, it is possible to provide a method for producing a peptide and glutathione, the method using, as a catalyst, a modified glutathione synthetase having an improved thermal stability as compared with wild-type glutathione synthetase, a gene encoding the modified glutathione synthetase, a transformant expressing the modified glutathione synthetase, and/or a treated material of the transformant.

Description of Embodiments

(1. Polypeptide)

A polypeptide in accordance with an embodiment of the present invention exhibits properties (a) to (b) or (c) to (d) below:

(a) being capable of carrying out a reaction of binding glycine to γ-glutamyl dipeptide; and
(b) having a higher thermal stability and/or a higher storage stability as compared with glutathione synthetase consisting of an amino acid sequence of SEQ ID NO: 1 shown in the Sequence Listing.
(c) being capable of producing GSH and/or GSSG; and
(d) having a higher thermal stability and/or a higher storage stability as compared with glutathione synthetase consisting of the amino acid sequence of SEQ ID NO: 1 shown in the Sequence Listing.

As used herein, "y-glutamyl dipeptide" means a compound in which, to a carboxyl group at a γ-position of glutamic acid, another amino acid is bound. Examples of the amino acid bound to the γ-position of the glutamic acid encompass: a neutral amino acid such as glycine, alanine, valine, leucine, isoleucine, serine, threonine, cysteine, methionine, asparagine, glutamine, and proline; an acidic amino acid such as asparagine acid and glutamic acid; a basic amino acid such as lysine, arginine, and histidine; an aromatic amino acid such as phenylalanine, tyrosine, and tryptophan; norvaline; norleucine; tert-leucine; hydroxyproline; α-aminobutyric acid; and β-aminobutyric acid.

A polypeptide in accordance with an embodiment of the present invention is a polypeptide which exhibits both the properties of: being capable of carrying out a reaction of binding glycine to γ-glutamyl dipeptide; and having a higher thermal stability and/or a higher storage stability as compared with glutathione synthetase consisting of the amino acid sequence of SEQ ID NO: 1 shown in the Sequence Listing. The polypeptide produces GSH and GSSG in a case where γ-glutamyl cysteine and oxidized γ-glutamyl cysteine are used as substrates, produces GSH in a case where γ-glutamyl cysteine is used as a substrate, and produces GSSG in a case where oxidized γ-glutamyl cysteine is used as a substrate. A polypeptide in accordance with an embodiment of the present invention is a polypeptide which exhibits both the properties of: being capable of producing GSH and GSSG; and having higher thermal stability and/or higher storage stability as compared with glutathione synthetase consisting of the amino acid sequence of SEQ ID NO: 1 shown in the Sequence Listing. The polypeptide produces GSH and GSSG in a case where γ-glutamyl cysteine and oxidized γ-glutamyl cysteine are used as substrates, produces GSH in a case where γ-glutamyl cysteine is used as a substrate, and produces GSSG in a case where oxidized γ-glutamyl cysteine is used as a substrate. A polypeptide in accordance with an embodiment of the present invention is a polypeptide which exhibits both the properties of: being capable of producing GSH; and having a higher thermal stability and/or a higher storage stability as compared with glutathione synthetase consisting of the amino acid sequence of SEQ ID NO: 1 shown in the Sequence Listing. The polypeptide produces GSH in a case where γ-glutamyl cysteine is used as a substrate. A polypeptide in accordance with an embodiment of the present invention is a polypeptide which exhibits both the properties of: being capable of producing GSSG; and having a higher thermal stability and/or a higher storage stability as compared with glutathione synthetase consisting of the amino acid sequence of SEQ ID NO: 1 shown in the Sequence Listing. The polypeptide produces GSSG in a case where oxidized γ-glutamyl cysteine is used as a substrate.

[Representation of mutation]

Amino acids, peptides, and proteins are herein represented with use of abbreviations below employed by IUPAC-IUB Commission of Biochemical Nomenclature (CBN). Unless otherwise specified, a sequence of an amino acid residue of each of a peptide and a protein is written such that a left end and a right end of the sequence represent an N-terminus and a C-terminus, respectively. For easy reference, the following nomenclature, which is generally used, is applied. According to the nomenclature, amino acid mutation is represented as "the original amino acid; position; substituted amino acid". For example, substitution of leucine at position 13 to serine is represented as "L13S". Multiple mutation is represented with use of a sign "/" to divide one mutation from another. For example, "S20T/E215D" represents substitution of serine at position 20 to threonine and substitution of glutamic acid at position 215 to asparagine acid.

[Abbreviations of amino acids]

A = Ala = alanine, C = Cys = cysteine,
D = Asp = asparagine acid, E = Glu = glutamic acid,

F = Phe = phenylalanine, G = Gly = glycine,
H = His = histidine, I = Ile = isoleucine,
K = Lys = lysine, L = Leu = leucine,
M = Met = methionine, N = Asn = asparagine,
P = Pro = proline, Q = Gln = glutamine,
R = Arg = arginine, S = Ser = serine,
T = Thr = threonine, V = Val = valine,
W = Trp = tryptophan, and Y = Tyr = tyrosine.

[Sequence identity]

"Sequence identities" of a polypeptide and a polynucleotide are each represented by a numerical value obtained by (i) aligning two polypeptides to be compared or two polynucleotides to be compared, (ii) dividing the number of amino acid positions or nucleic acid base positions (e.g., A, T, C, G, U, or I) coinciding between the two sequences by the total number of compared bases, and (iii) multiplying a result of the division by 100.

A sequence identity can be calculated, for example, with use of the following sequence analysis tools: GCG Wisconsin Package (University of Wisconsin); the ExPASy World Wide Web molecular biology server (Swiss Institute of Bioinfomatics); BLAST (National Center for Biotechnology Information, U.S.); and GENETYX (GENETYX CORPORATION).

In an embodiment of the present invention, wild-type glutathione synthetase in which mutation has not been introduced (also referred to herein as "wild-type enzyme") is a polypeptide which consists of 314 amino acid residues of SEQ ID NO: 1 shown in the Sequence Listing and has an ability to produce γ-Glu-X-Gly (e.g., γ-glutamyl-alanyl-glycine) from γ-glutamyl dipeptide represented by γ-Glu-X (e.g., γ-glutamyl alanine) and glycine, an ability to produce GSH from γ-glutamyl cysteine and glycine, or an ability to produce GSSG from oxidized γ-glutamyl cysteine and glycine.

The polypeptide is not limited to a specific origin, but the polypeptide is glutathione synthetase that is preferably derived from a microorganism belonging to the family *Hydrogenophilales*, more preferably derived from a microorganism belonging to the genus *Thiobacillus*, even more preferably derived from *Thiobacillus denitrificans* ATCC25259 strain.

In an embodiment of the present invention, wild-type glutathione synthetase is encoded by a polynucleotide. The polynucleotide is not specifically limited as long as it encodes amino acids of SEQ ID NO. 1 shown in the Sequence Listing. For example, the polynucleotide is a polynucleotide of SEQ ID NO: 2 shown in the Sequence Listing. The polynucleotide can be obtained from the family *Hydrogenophilales*, more preferably from the genus *Thiobacillus*, and even more preferably from *Thiobacillus denitrificans* ATCC25259 strain in accordance with a general genetic engineering method described in Molecular Cloning 2nd Edition (Joseph Sambrook, Cold Spring Harbor Laboratory Press (1989)) or the like. Further, a polynucleotide encoding the amino acid sequence of SEQ ID NO. 1, as represented by SEQ ID NO: 3 shown in the Sequence Listing, can be obtained by chemical synthesis, and a polynucleotide which has been subjected to codon optimization in accordance with a host can be obtained by chemical synthesis.

That is, by carrying out PCR with use of a genomic DNA of *Thiobacillus denitrificans* ATCC25259 strain as a template, a polynucleotide encoding the amino acid sequence of SEQ ID NO: 1 or a polynucleotide of SEQ ID NO: 2 can be amplified so as to prepare a wild-type enzyme gene, and a polynucleotide encoding the amino acid sequence of SEQ ID NO: 1 (for example, a polynucleotide of SEQ ID NO: 3) can be chemically synthesized.

A polypeptide in accordance with an embodiment of the present invention may be obtained by making a modification to the amino acid sequence of SEQ ID NO. 1.

The modification made to the amino acid sequence of SEQ ID NO: 1 may be substitution, addition, insertion, or deletion. The modification may include only one type of modification (e.g., substitution) or may include two or more types of modification (e.g., substitution and insertion). The "plurality of amino acids" means, for example, 63, preferably 47, more preferably 31, even more preferably 25, 16, 9, 7, 5, 4, 3, or 2 amino acids.

A sequence identity between an amino acid sequence to which a modification has been made and the amino acid sequence of SEQ ID NO: 1 is 80% or more, more preferably 85% or more, even more preferably 90% or more, more preferably 92% or more, 95% or more, 97% or more, 98% or more, 98.5% or more, or 99% or more.

A polypeptide in accordance with an embodiment of the present invention may be (i) a polypeptide which, in addition to exhibiting the properties of (a) to (b) or (c) to (d) or exhibiting a property of being capable of producing GSH and GSSG with use of γ-glutamyl cysteine and oxidized γ-glutamyl cysteine as substrates, a property of being capable of producing GSH with use of γ-glutamyl cysteine as a substrate, or a property of being capable of producing GSSG with use of oxidized γ-glutamyl cysteine as a substrate, is obtained by carrying out amino acid substitution, insertion, deletion, and/or addition, as described below, in the amino acid sequence of SEQ ID NO: 1 shown in the Sequence Listing or (ii) a polypeptide having a certain sequence identity with respect to such an amino acid sequence.

A polypeptide in accordance with an embodiment of the present invention is obtained by carrying out amino acid substitution, insertion, deletion, and/or addition at a position(s) not particularly limited in the amino acid sequence of SEQ ID NO: 1 shown in the Sequence Listing, and is preferably a polypeptide consisting of an amino acid sequence which is obtained by substitution of one or more amino acids in the amino acid sequence of SEQ ID NO: 1 shown in the Sequence Listing, the one or more amino acids being selected from the group consisting of amino acids at respective positions 13, 17, 20, 23, 39, 70, 78, 101, 113, 125, 126, 136, 138, 149, 152, 154, 155, 197, 200, 215, 226, 227, 230, 239, 241, 246, 249, 254, 260, 262, 263, 270, 278, 299, 305, 307, and 310. A polypeptide in accordance with another embodiment of the present invention may be a polypeptide having an amino acid sequence which is obtained by substitution of one or more amino acids at a position(s) selected from the above positions in the amino acid sequence of SEQ ID NO: 1 shown in the Sequence Listing, and in which one or more (e.g., 63, preferably 47, more preferably 31, even more preferably 25, 16, 9, 7, 5, 4, 3, or 2) amino acids at a position(s) other than the above positions are substituted, added, inserted, or deleted. A polypeptide in accordance with another embodiment of the present invention may be a polypeptide having an amino acid sequence which is obtained by substitution of one or more amino acids at a position(s) selected from the above positions in the amino acid sequence of SEQ ID NO: 1 shown in the Sequence Listing, and which has a sequence identity of 80% or more, preferably 85% or more, more preferably 90% or more, even more preferably 92% or more, 95% or more, 97% or more, 98% or more, 98.5% or more, or 99% or more with respect to the amino acid sequence of SEQ ID NO: 1 shown in the Sequence Listing except for said positions.

More preferably, a polypeptide in accordance with an embodiment of the present invention is a polypeptide which consists of an amino acid sequence which is obtained by substitution of one or more amino acids in the amino acid sequence of SEQ ID NO: 1 shown in the Sequence Listing, the substitution of the one or more amino acids being selected from the group consisting of: substitution of an amino acid at position 13 to serine; substitution of an amino acid at position 17 to glutamic acid; substitution of an amino acid at position 20 to threonine; substitution of an amino acid at position 23 to leucine; substitution of an amino acid at position 39 to threonine; substitution of an amino acid at position 70 to serine; substitution of an amino acid at position 78 to leucine; substitution of an amino acid at position 101 to asparagine, glutamine, serine, or threonine; substitution of an amino acid at position 113 to histidine; substitution of an amino acid at position 125 to valine; substitution of an amino acid at position 126 to asparagine; substitution of an amino acid at position 136 to threonine; substitution of an amino acid at position 138 to alanine; substitution of an amino acid at position 149 to glutamine; substitution of an amino acid at position 152 to glutamine; substitution of an amino acid at position 154 to asparagine; substitution of an amino acid at position 155 to leucine; substitution of an amino acid at position 197 to glutamine; substitution of an amino acid at position 200 to serine; substitution of an amino acid at position 215 to asparagine acid; substitution of an amino acid at position 226 to arginine; substitution of an amino acid at position 227 to serine; substitution of an amino acid at position 230 to proline; substitution of an amino acid at position 239 to serine; substitution of an amino acid at position 241 to histidine; substitution of an amino acid at position 246 to arginine; substitution of an amino acid at position 249 to glutamic acid; substitution of an amino acid at position 254 to asparagine acid; substitution of an amino acid at position 260 to alanine, cystein, glycine, glutamine, or threonine; substitution of an amino acid at position 262 to cysteine; substitution of an amino acid at position 263 to arginine; substitution of an amino acid at position 270 to isoleucine; substitution of an amino acid at a position 278 to glycine or alanine; substitution of an amino acid at position 299 to alanine; substitution of an amino acid at position 305 to glycine; substitution of an amino acid at position 307 to valine; and substitution of an amino acid at position 310 to threonine. A polypeptide in accordance with another embodiment of the present invention may be a polypeptide having an amino acid sequence which is obtained by substitution of one or more amino acids at a position(s) selected from the above positions in the amino acid sequence of SEQ ID NO: 1 shown in the Sequence Listing, and in which one or more (e.g., 63, preferably 47, more preferably 31, even more preferably 25, 16, 9, 7, 5, 4, 3, or 2) amino acids at a position(s) other than the above positions are substituted, added, inserted, or deleted. A polypeptide in accordance with another embodiment of the present invention may be a polypeptide having an amino acid sequence which is obtained by substitution of one or more amino acids at a position(s) selected from the above positions in the amino acid sequence of SEQ ID NO: 1 shown in the Sequence Listing, and which has a sequence identity of 80% or more, preferably 85% or more, more preferably 90% or more, even more preferably 92% or more, 95% or more, 97% or more, 98% or more, 98.5% or more, or 99% or more with respect to the amino acid sequence of SEQ ID NO: 1 shown in the Sequence Listing except for said positions.

More preferably, a polypeptide in accordance with an embodiment of the present invention is a polypeptide which is obtained by amino acid substitution in the amino acid sequence of SEQ ID NO: 1 shown in the Sequence Listing, the amino acid substitution being represented by any one of (1) to (35) below:

(1) substitution of an amino acid at position 13 to serine;
(2) substitution of an amino acid at position 17 to glutamic acid, an amino acid at position 113 to histidine, and an amino acid at position 230 to proline;
(3) substitution of an amino acid at position 20 to threonine and an amino acid at position 215 to asparagine acid;
(4) substitution of an amino acid at position 20 to threonine and an amino acid at position 241 to histidine;
(5) substitution of an amino acid at position 23 to leucine and an amino acid at position 126 to asparagine;

(6) substitution of an amino acid at position 39 to threonine and an amino acid at position 260 to alanine;
(7) substitution of an amino acid at position 70 to serine and an amino acid at position 260 to alanine;
(8) substitution of an amino acid at position 78 to leucine and an amino acid at position 278 to alanine;
(9) substitution of an amino acid at position 101 to asparagine;
(10) substitution of an amino acid at position 101 to glutamine;
(11) substitution of an amino acid at position 101 to serine;
(12) substitution of an amino acid at position 101 to serine and an amino acid at position 260 to alanine;
(13) substitution of an amino acid at position 101 to threonine;
(14) substitution of an amino acid at position 125 to valine and an amino acid at position 249 to glutamic acid;
(15) substitution of an amino acid at position 125 to valine and an amino acid at position 152 to glutamine;
(16) substitution of an amino acid at position 136 to threonine;
(17) substitution of an amino acid at position 138 to alanine, an amino acid at position 149 to glutamine, an amino acid at position 241 to histidine, and an amino acid at position 263 to glutamine;
(18) substitution of an amino acid at position 154 to asparagine and an amino acid at position 246 to arginine;
(19) substitution of an amino acid at position 155 to leucine and an amino acid at position 239 to serine;
(20) substitution of an amino acid at position 197 to glutamine;
(21) substitution of an amino acid at position 200 to serine and an amino acid at position 260 to alanine;
(22) substitution of an amino acid at position 226 to arginine and an amino acid at position 260 to alanine;
(23) substitution of an amino acid at position 227 to serine and an amino acid at position 260 to alanine;
(24) substitution of an amino acid at position 254 to asparagine acid and an amino acid at position 260 to alanine;
(25) substitution of an amino acid at position 260 to alanine;
(26) substitution of an amino acid at position 260 to alanine, an amino acid at position 278 to glycine, and an amino acid at position 307 to valine;
(27) substitution of an amino acid at position 260 to alanine and an amino acid at position 299 to alanine;
(28) substitution of an amino acid at position 260 to alanine and an amino acid at position 305 to glycine;
(29) substitution of an amino acid at position 260 to alanine and an amino acid at position 310 to threonine;
(30) substitution of an amino acid at position 260 to cysteine;
(31) substitution of an amino acid at position 260 to glycine;
(32) substitution of an amino acid at position 260 to glutamine;
(33) substitution of an amino acid at position 260 to threonine;
(34) substitution of an amino acid at position 262 to cysteine; and
(35) substitution of an amino acid at position 270 to isoleucine. A polypeptide in accordance with another embodiment of the present invention may be a polypeptide which is obtained by amino acid substitution at a position(s) selected from the above positions in the amino acid sequence of SEQ ID NO: 1 shown in the Sequence Listing, and in which one or more (e.g., 63, preferably 47, more preferably 31, even more preferably 25, 16, 9, 7, 5, 4, 3, or 2) amino acids at a position(s) other than the above positions are substituted, added, inserted, or deleted. A polypeptide in accordance with another embodiment of the present invention may be a polypeptide which is obtained by amino acid substitution at a position(s) selected from the above positions in the amino acid sequence of SEQ ID NO: 1 shown in the Sequence Listing, and which has a sequence identity of 80% or more, preferably 85% or more, more preferably 90% or more, even more preferably 92% or more, 95% or more, 97% or more, 98% or more, 98.5% or more, or 99% or more with respect to the amino acid sequence of SEQ ID NO: 1 shown in the Sequence Listing except for said positions.

In an embodiment of the present invention, a thermal stability of an enzyme can be evaluated, for example, by the following method.

(Method for evaluating thermal stability of enzyme)

A cell-free extract containing an enzyme is incubated at a given temperature (e.g., 40°C to 90°C) for a given length of time (e.g., 0.1 minute to 48 hours). Samples of the cell-free extract, one of which has not been subjected to any heat treatment and the other of which has been subjected to a heat treatment, are each diluted with 0.01 M to 1.0 M Tris-HCl buffer solution (pH: 6 to 9). With use of a resultant diluted solution, enzyme activity measurement is carried out in accordance with [Glutathione synthetase activity evaluation method (1)] or [Glutathione synthetase activity evaluation method (2)] below, so that a remaining activity after the heat treatment can be calculated in accordance with a formula below. This remaining activity is used as an index of thermal stability.

$$\text{Remaining activity (\%)} = [\text{an enzyme activity of the sample subjected to the heat treatment}] \div [\text{an enzyme activity of the sample not subjected to any heat treatment}] \times 100$$

[Glutathione synthetase activity evaluation method (1)]

A reaction solution, which contains 1 mM to 50 mM substrate (e.g., γ-glutamyl cysteine, oxidized γ-glutamyl cysteine), 30 mM ATP disodium salt, 30 mM glycine, 10 mM magnesium sulfate heptahydrate, and a polypeptide in accordance with an embodiment of the present invention in 200 mM Tris-HCl buffer solution (pH: 8.5), is reacted at 30°C, and a resultant reaction solution is subjected to HPLC analysis to quantify a product (e.g., GSH, GSSG, γ-glutamyl-cysteinylglutathione). This enables evaluation of glutathione synthetase activity. A glutathione synthetase activity 1 U is defined as an amount of the enzyme which catalyzes a reaction of binding 1 pmol of glycine to a substrate per minute.

(HPLC condition)

Column: Develosil ODS-HG-3 (diameter 4.6 mm × 250 mm, manufactured by Nomura Chemical Co., Ltd.)
Eluent: a liquid obtained by dissolving 12.2 g of potassium dihydrogen phosphate and 7.2 g of sodium 1-heptanoate in 1.8 L of distilled water, subsequently adjusting a resultant solution to pH 3.0, and adding and dissolving 100 ml of methanol in the solution
Flow rate: 1.0 ml/min
Column temperature: 40°C
Measurement wavelength: 210 nm

[Glutathione synthetase activity evaluation method (2)]

Measurement of glutathione synthetase activity can be carried out also by using a less inexpensively available compound (e.g., γ-glutamyl alanine) as an alternative substrate in place of γ-glutamyl cysteine or oxidized γ-glutamyl cysteine. More specifically, the alternative substrate is used in place of γ-glutamyl cysteine and/or oxidized γ-glutamyl cysteine in the above method. Activity measurement with use of the alternative substrate is carried out in accordance with the following method. A reaction solution, which contains 1 mM to 50 mM substrate, 20 mM ATP disodium salt, 20 mM glycine, 10 mM magnesium sulfate heptahydrate, and a polypeptide in accordance with the present invention in 200 mM Tris-HCl buffer solution (pH: 8.5) is reacted at 30°C, and a resultant reaction solution is subjected to HPLC analysis to quantify a product. This enables evaluation of an activity of the enzyme. An enzyme activity 1 U is defined as an amount of the enzyme which catalyzes production of 1 μmol of the product per minute.

(HPLC condition)

Column: SUMICHIRAL OA-5000 (diameter 4.6 mm × 250 mm, manufactured by Sumika Chemical Analysis Service, Ltd.)
Eluent: a liquid obtained by dissolving 2 mM copper sulfate in a solution of distilled water : isopropyl alcohol = 95:5
Flow rate: 1.0 ml/min
Column temperature: 40°C
Measurement wavelength: 254 nm

As used herein, "have an improved thermal stability" means that a remaining activity measured in a case where the above-described evaluation is carried out is higher than that of glutathione synthetase of SEQ ID NO: 1 shown in the Sequence Listing by 1% or more, preferably 5% or more, more preferably 10% or more, most preferably 20% or more.
Specifically, "have an improved thermal stability" means that in a case where a remaining activity with respect to γ-glutamyl cysteine, oxidized γ-glutamyl cysteine, or γ-glutamyl alanine after incubation at 60°C or 70°C is measured by a method described later in Reference Example 3 or 4, at least one of the remaining activities measured by the respective methods of Reference Examples 3 and 4 is higher than that of a wild-type enzyme by 1% or more, preferably 5% or more, more preferably 10% or more, most preferably 20% or more.

Since an enzyme undergoes denaturation by heat, an enzyme having a high thermal stability generally has a highly stable enzyme activity under a slow temperature condition. Thermal stability is a function representing stability, including those of hydrogen bonding, hydrophobic bonding, ion interaction, metal bonding, and/or disulfide bonding. Such a stability effect contributes to long-term stability of an enzyme (Pure & Appl. Chem., 63, 10, 1527-1540 (1991)). That is, the higher the thermal stability of an enzyme is, the higher the storage stability of the enzyme tends to be (in other words, thermal stability and storage stability are correlated). Therefore, glutathione synthetase in accordance with an embodiment of the present invention has not only an excellent thermal stability but also an excellent storage stability.

As used herein, "have a high storage stability" means that, for example, in a case where the enzyme, a solution containing the enzyme, or a recombinant producing the enzyme is allowed to stand at 4°C to 40°C for a long time (e.g., 1 hour to 2 years) and then is subjected to the above-described activity measurement, a ratio of an activity of the enzyme before allowing to stand to an activity of the enzyme after allowing to stand is higher than that of a wild-type enzyme by 1% or more, preferably 5% or more, more preferably 10% or more, most preferably 20% or more.

Glutathione synthetase in accordance with an embodiment of the present invention can be searched for in accordance with the following method.

Specifically, with use of a kit based on error-prone PCR (Leung et al., Technique 1, 11-15 (1989)) or a similar principle, a DNA fragment which is formed by introducing substitution, insertion, deletion, and/or addition of one or more base sequences in a base sequence (a wild-type enzyme gene which has been chemically synthesized) of SEQ ID NO: 3 shown in the Sequence Listing can be obtained. For example, by using the wild-type enzyme gene as a template as well as a primer 1 (5'-GGGTTTCATATGAAACTGCTGTTCGTCG-3' (SEQ ID NO. 4 shown in the Sequence Listing)), a primer 2 (5'-CCGGAATTCTTATCATTCCGGACGCG-3' (SEQ ID NO: 5 shown in the Sequence Listing)), and Diversify PCR Random Mutagenesis Kit (manufactured by Clontech), a plurality of kinds of double-stranded DNAs (mutated enzyme genes), each of which is formed by randomly introducing mutation over a full length of a gene encoding the wild-type enzyme, adding an NdeI recognition site to a start codon, and adding an EcoRI recognition site to a position immediately after a stop codon, can be obtained. The amplified fragments thus obtained are each digested with use of NdeI and EcoRI and inserted between an NdeI recognition site and an EcoRI recognition site downstream of a lac promoter of a plasmid pUCN18 (a plasmid obtained by modifying T at position 185 of pUC18 (manufactured by Takara-Bio Inc.) to A by means of PCR so as to destroy an NdeI site and further modifying GC at positions 471-472 to TG so as to newly introduce an NdeI site). With use of this plasmid, *Escherichia coli* HB101 strain (hereinafter referred to as *E. coli* HB101) is transformed. The transformed *E. coli* is spread on an LB plate medium containing 100 μg/mL of ampicillin. Thus obtained is a single colony of *E. coli.* Further, with use of a mutated enzyme gene obtained by the above-described method in place of the wild-type gene, mutation can be further introduced to the mutated enzyme gene by a similar operation so as to construct a mutated enzyme gene library.

From the library, a modified glutathione synthetase in accordance with an embodiment of the present invention can be selected. The method of selection is not particularly limited, but is preferably a method shown below. Note that a modified enzyme (or a modified glutathione synthetase) is a mutated enzyme into which mutation for imparting a desired property has been introduced. As used herein, a modified enzyme means glutathione synthetase which is selected from the mutated enzyme gene library as having a higher thermal stability and/or a higher storage stability as compared with a wild-type enzyme.

[Method for selecting enzyme with improved thermal stability by plate evaluation]

Recombinant bacteria of the mutated enzyme gene library and recombinant bacteria producing a wild-type enzyme (e.g., *E. coli* HB101 (pTDGSH2) shown in Reference Example 3) are each inoculated onto an appropriate medium (e.g., a 2 × YT medium (tryptone: 1.6%, yeast extract: 1.0%, sodium chloride: 0.5%, pH: 7.0) containing 200 μg/ml of ampicillin), and is subjected to shake culture at 37°C for 24 hours. Each culture solution thus obtained is subjected to centrifugal separation to remove a supernatant, and is suspended in an appropriate buffer solution (e.g., 0.2 M of a Tris-HCl buffer solution (pH: 8.5)). This suspension is disrupted, and then subjected to centrifugation so as to remove a precipitate. Thus obtained are cell-free extracts. The cell-free extracts containing respective enzymes are each heated at an appropriate temperature (preferably 40°C to 80°C) so as to be incubated. After approximately 0.1 minute to 48 hours of incubation, each cell-free extract is dispensed onto a 96-well plate (manufactured by AGC TECHNO GLASS CO., LTD.), and a Tris-HCl buffer solution (pH: 5 to 9) containing ATP disodium salt (preferably 30 mM), magnesium sulfate heptahydrate (preferably 10 mM), a solution containing oxidized γ-glutamyl cysteine (preferably 15 mM), and glycine (preferably 30 mM) is added so as to carry out incubation at 10°C to 50°C for 3 minutes to 48 hours. A method for quantifying glutathione produced in the reaction solution can be, for example, the following method. The reaction solution is dispensed onto another 96-well plate (manufactured by AGC TECHNO GLASS CO., LTD.), and a Tris-HCl buffer solution (pH: 5 to 9) containing glutathione reductase (preferably 30 U/L or more, manufactured by Sigma-Aldrich) and NADPH (preferably 1.2 mM) is add so as to carry out incubation at 10°C to 50°C for 0.1 minute to 60 minutes. In this process, oxidized glutathione produced by glutathione synthetase is converted into reduced glutathione. To this, a Tris-HCl buffer solution (pH: 5 to 9) containing (preferably 0.2 mg/mL of) 5,5'-dithiobis(2-nitrobenzonate) (hereinafter referred to as "DTNB") is added, and visual observation and detection of absorption of light at 405 nm are carried out over time. At this time, in a case where reduced glutathione is present in the reaction solution, absorption of light at 405 nm is detectable. A ratio of light absorption of a sample obtained by a glutathione synthesis reaction with use of a cell-free extract that has been subjected to a heat treatment to light absorption of a control obtained by a glutathione synthesis reaction with use of a cell-free extract that has not been subjected to a heat treatment is defined as an activity residual rate. A sample whose activity residual rate is higher than that of wild-type glutathione synthetase is selected as an enzyme having an improved thermal stability. Plasmids are extracted from the culture solution of the enzyme thus selected, and with use of BigDye Terminator Cycle Sequencing Kit (manufactured by Applied Biosystems Japan, Ltd.) and Applied Biosystems 3130xl Genetic Analyzer (manufactured by Applied Biosystems Japan, Ltd.), the base sequence of a modified glutathione synthetase gene is determined, so that identification of a mutation site(s) is made possible.

Among mutations included in a plurality of modified glutathione synthetase genes obtained, some of the mutations included in one modified glutathione synthetase gene are combined together in the other modified glutathione synthetase gene or the mutations included in the plurality of modified glutathione synthetase genes are combined together by site-specific mutation introduction, so that a modified glutathione synthetase having an enhanced thermal stability can be prepared. Such a modified glutathione synthetase is also encompassed in the scope of a polypeptide in accordance with an embodiment of the present invention.

(2. Polynucleotide)

In an embodiment of the present invention, a polynucleotide encoding the above-described polypeptide in accordance with an embodiment of the present invention is provided.

A polynucleotide in accordance with an embodiment of the present invention is not particularly limited, provided that the polynucleotide encodes the above-described polypeptide in accordance with an embodiment of the present invention. Examples of a polynucleotide in accordance with an embodiment of the present invention encompass: a polynucleotide consisting of a base sequence encoding a wild-type enzyme of SEQ ID NO: 2 or 3 shown in the Sequence Listing; and a polynucleotide obtained by modifying the polynucleotide.

A method for modifying a wild-type enzyme gene may be a well-known method described in Current Protocols in Molecular Biology (Frederick M. Ausubel, Greene Publishing Associates and Wiley-Interscience (1989)) or the like. That is, by substituting, adding, inserting, or deleting one base or a plurality of bases (e.g., 40, preferably 20, more preferably 10, even more preferably 5, 4, 3, or 2 bases) of a wild-type enzyme gene, it is possible to prepare a polynucleotide which is formed by modifying an amino acid sequence of a wild-type enzyme. Examples of the method for modifying a wild-type enzyme gene encompass: a mutation introduction method using PCR such as error-prone PCR (Leung et al., Technique 1, 11-15 (1989)) or the like; and use of a commercially available kit such as Diversify PCR Random Mutagenesis Kit (manufactured by Clontech), Transformer Mutagenesis Kit (manufactured by Clontech), EXOIII/Mung Bean Deletion Kit (manufactured by Stratagene), QuickChange Site Directed Mutagenesis Kit (manufactured by Stratagene) and the like.

In a case of preparing a polynucleotide by a site-specific mutation introduction method, examples of the site-specific mutation introduction encompass methods according to Olfert Landt et al. (Gene, 96, 125-128 (1990)), Smith et al. (Genetic Engineering, 3, 1, Setlow, J. Plenum Press), Vlasuk et al. (Experimental Manipulation of Gene Expression, Inouye, M. Academic Press), and Hos. N. Hunt et al. (Gene, 77, 51 (1989)); use of a commercially available kit QuikChange II Kit (manufactured by Stratagene); and the like. In a case of introducing mutation at two positions, a method in accordance with the above method can be repeated twice to obtain a desired polynucleotide in accordance with an embodiment of the present invention. Note that also in a case where a plurality of other positions are substituted by other amino acids, the above method can be carried out to obtain a desired polynucleotide in accordance with an embodiment of the present invention.

A polynucleotide encoding a polypeptide in accordance with an embodiment of the present invention is, for example, preferably a polynucleotide which encodes a polypeptide that has (i) an activity of producing reduced glutathione, oxidized glutathione, or γ-glutamyl-alanyl-glycine with use of glycin and each of γ-glutamyl cysteine, oxidized γ-glutamyl cysteine, and γ-glutamyl alanine and (ii) a higher thermal stability as compared with glutathione synthetase consisting of the amino acid sequence of SEQ ID NO: 1 shown in the Sequence Listing and which hybridizes under stringent conditions with a polynucleotide including a base sequence complementary to a polynucleotide consisting of a base sequence of SEQ ID NO: 2 or 3.

Note here that "a polynucleotide which hybridizes under stringent conditions with a polynucleotide consisting of a base sequence complementary to a polynucleotide of SEQ ID NO: 2" means a polynucleotide which is obtained by colony hybridization, plaque hybridization, Southern hybridization, or the like under stringent conditions with use of, as a probe, a polynucleotide consisting of a base sequence complementary to a base sequence of SEQ ID NO: 2 shown in the Sequence Listing.

Hybridization can be carried out, for example, in accordance with a method described in Molecular Cloning 2nd Edition (Joseph Sambrook, Cold Spring Harbor Laboratory Press (1989)) or the like. Note that examples of "a polynucleotide which hybridizes under stringent conditions" encompass a DNA which can be obtained by carrying out hybridization at 65°C in the presence of 0.7 M to 1.0 M of sodium chloride with use of a filter to which a colony-derived or plaque-derived polynucleotide has been fixed and then washing the filter under a condition of 65°C with use of 0.3 × SSC (1 × SSC consists of 150 mM sodium chloride and 15 mM citric acid sodium). The "polynucleotide which hybridizes under stringent conditions" is a polynucleotide which can be obtained preferably by washing at 65°C with use of 0.13 × SSC, even more preferably by washing at 65°C with use of 0.09 × SSC, particularly preferably by washing at 65°C with use of 0.07, 0.06, 0.04, 0.03, or 0.02 × SSC.

The present invention is not particularly limited to the hybridization conditions described above. A plurality of factors such as temperature and salt concentration are possible factors which affect stringency of hybridization. A person skilled in the art can achieve optimum stringency by making appropriate selections with respect to these factors.

Examples of a polynucleotide capable of hybridizing under the above conditions encompass a DNA having a sequence identity of preferably 78% or more, more preferably 84% or more, even more preferably 87% or more, particularly preferably 89% or more, 90% or more, 93% or more, 95% or more, or 97% or more with respect to a polynucleotide of SEQ ID NO: 2. Such a polynucleotide is encompassed within the scope of the above-described polynucleotide in accordance with an embodiment of the present invention provided that a polypeptide encoded by the polynucleotide has polypeptide properties in accordance with an embodiment of the present invention.

Note that examples of "a polynucleotide which hybridizes under stringent conditions with a polynucleotide consisting of a base sequence complementary to a polynucleotide of SEQ ID NO: 3 shown in the Sequence Listing", too, similarly encompass a DNA which can be obtained by carrying out hybridization at 65°C in the presence of 0.7 M to 1.0 M of sodium chloride and then washing the filter under a condition of 65°C with use of 0.6 × SSC (1 × SSC consists of 150 mM sodium chloride and 15 mM citric acid sodium). The "polynucleotide which hybridizes under stringent conditions" is a polynucleotide which can be obtained preferably by washing at 65°C with use of 0.25 × SSC, even more preferably by washing at 65°C with use of 0.15 × SSC, particularly preferably by washing at 65°C with use of 0.12, 0.10, 0.07, 0.05, or 0.04 × SSC.

The present invention is not particularly limited to the hybridization conditions described above. A plurality of factors such as temperature and salt concentration are possible factors which affect stringency of hybridization. A person skilled in the art can achieve optimum stringency by making appropriate selections with respect to these factors.

Examples of a polynucleotide capable of hybridizing under the above conditions encompass a DNA having a sequence identity of preferably 78% or more, more preferably 84% or more, even more preferably 87% or more, particularly preferably 89% or more, 90% or more, 93% or more, 95% or more, or 97% or more with respect to a polynucleotide of SEQ ID NO: 2. Such a polynucleotide is encompassed within the scope of a polynucleotide in accordance with the above embodiment provided that a polypeptide encoded by the polynucleotide has polypeptide properties in accordance with an embodiment of the present invention.

(3. Transformant)

In an embodiment of the present invention, a transformant expressing the above-described polypeptide in accordance with an embodiment of the present invention or the above-described polynucleotide in accordance with an embodiment of the present invention is provided.

A polynucleotide expression vector can be prepared by inserting, into an expression vector, a polynucleotide encoding a polypeptide in accordance with an embodiment of the present invention.

As used herein, an "expression vector" means a vector which includes a sequence of a promoter or the like and is constructed so that a gene is expressed in a cell which has been transformed. The expression vector used above is not particularly limited provided that the expression vector is capable of expressing a polypeptide encoded by the above-described polynucleotide in accordance with an embodiment of the present invention in an appropriate host organism.

As used herein, a "vector" means a nucleic acid molecule into which a gene is integrated and in which a recombinant DNA is amplified, maintained, or introduced. Examples of such a vector encompass a plasmid vector, a phage vector, a cosmid vector, and the like. Further, a shuttle vector which is capable of exchanging a gene with another host strain can also be used as the above vector.

A vector in accordance with an embodiment of the present invention may include a regulator which is operably linked to a polynucleotide in accordance with an embodiment of the present invention. As used herein, a "regulator" means a base sequence which has a functional promoter and an optional associated transcription element (e.g., an enhancer, a CCAAT box, a TATA box, an SPI site, or the like). As used herein, "operably linked" means that a regulation element of various kinds (including the regulator), which regulates an expression of a gene and is exemplified by a promoter, an enhancer, or the like, and a gene are linked to each other such that the gene and the regulation element can operate within a host cell. It is a matter known to a person skilled in the art that the type and kind of a regulator can vary depending on a host organism into which a vector in accordance with an embodiment of the present invention is introduced.

In an embodiment of the present invention, for example, in a case where a host organism is *E. coli,* a vector in accordance with an embodiment of the present invention typically includes a regulator such as a lacUV5 promoter, a trp promoter, a trc promoter, a tac promoter, a lpp promoter, a tufB promoter, a recA promoter, or a pL promoter and can be suitably used as an expression vector including an expression unit which is operably linked to a polynucleotide in accordance with an embodiment of the present invention. Examples of such a vector encompass pUCN18 (see Reference Example 1), pSTV28 (manufactured by Takara-Bio Inc.), pUCNT (International Publication No. 94/03613), and the like.

A vector, a promoter, and the like which can be used in each host organism are described in detail in Biseibutsugaku Kiso Koza (8, Ando Tadahiko, KYORITSU SHUPPAN (1987)) and the like.

A vector in accordance with an embodiment of the present invention may further include a polynucleotide encoding a polypeptide having an ATP regeneration ability. Examples of the polypeptide having an ATP regeneration ability encompass polyphosphate kinase (hereinafter also referred to as "PPK"), adenylate kinase, pyruvate kinase, acetate kinase, and phosphocreatine kinase.

By transforming a host cell with use of a vector in accordance with an embodiment of the present invention, a transformant in accordance with an embodiment of the present invention can be obtained. The transformant in accordance with the embodiment of the present invention also encompass a transformant which is obtained by introducing, into a chromosome, a polynucleotide encoding a polypeptide in accordance with an embodiment of the present invention.

A host cell transformed by introduction of a vector in accordance with an embodiment of the present invention is not particularly limited provided that the host cell is a cell which can be transformed by a polynucleotide expression vector including a polynucleotide encoding each polypeptide in accordance with an embodiment of the present invention and in which the polypeptide encoded by the polynucleotide introduced can be expressed. Examples of a microorganism which is usable as a host cell encompass: bacteria for which a host vector system has been developed, belonging to the genus *Escherichia*, the genus *Bacillus*, the genus *Pseudomonas*, the genus *Serratia*, the genus *Brevibacterium*, the genus *Corynebacterium*, the genus Streptococcus, the genus *Lactobacillus*, and the like; actinomycetes for which a host vector system has been developed, belonging to the genus *Rhodococcus*, the genus *Streptomyces*, and the like; yeasts for which a host vector system has been developed, belonging to the genus *Saccharomyces*, the genus *Kluyveromyces*, the genus *Schizosaccharomyces*, the genus *Zygosaccharomyces*, the genus *Yarrowia*, the genus *Trichosporon*, the genus *Rhodosporidium*, the genus *Pichia*, the genus *Candida*, and the like; fungi for which a host vector system has been developed, belonging to the genus *Neurospora*, the genus *Aspergillus*, the genus *Cephalosporium*, the genus *Trichoderma*, and the like; and the like. Further, various host vector systems have been developed for plants and animals apart from microorganisms. In particular, systems which cause a foreign protein to be expressed in large quantity in an insect that uses a silkworm (Nature, 315, 592-594 (1985)) and a plant such as rape, corn, and potato have been developed and can be suitably used. Among these, the bacteria are preferable from the viewpoint of introduction efficiency and expression efficiency, and *E. coli* is particularly preferable.

A vector in accordance with an embodiment of the present invention can be introduced into a host cell in accordance with a well-known method. For example, in a case where a plasmid (pNKPm 01 to pNKPm 35 shown in Examples 1, 2, 7, 8, and 12 to 18) in accordance with an embodiment of the present invention obtained by introducing a polynucleotide encoding a modified glutathione synthetase into the above-described expression vector pUCN18 is used as a polynucleotide expression vector and *E. coli* is used as a host microorganism, a transformant (e.g., *E.coli* HB101 (pTDGSH2m35) shown in Example 18) formed by introduction of the vector into a host cell can be obtained by using a commercially available *E.coli* HB101 competent cell (manufactured by Takara-Bio Inc.) or the like and carrying out an operation in accordance with a protocol of the *E.coli* HB101 competent cell or the like.

Further, it is possible to prepare a transformant in which both a polypeptide in accordance with an embodiment of the present invention and the above-described polypeptide having an ATP regeneration ability are expressed within the same bacterial cell. That is, the transformant can be obtained by (i) incorporating, into the same vector, a polynucleotide encoding a polypeptide in accordance with an embodiment of the present invention and a polynucleotide encoding the polynucleotide having an ATP regeneration ability and (ii) introducing the vector into a host cell. Further, the transformant can be obtained also by (i) incorporating these two kinds of DNAs respectively into two kinds of vectors belonging to different incompatibility groups and (ii) introducing the two vectors thus obtained into the same host cell.

Examples of the transformant thus obtained encompass, but not limited to, a transformant which is obtained by introducing, into an *E.coli* HB101 competent cell (manufactured by Takara-Bio Inc.), (i) a recombinant vector (e.g., pTDGSH2m15 shown in Example 2) obtained by introducing, into the above-described expression vector pUCN18, a nucleotide encoding a modified glutathione synthetase in accordance with an embodiment of the present invention and (ii) a vector including a polynucleotide encoding polyphosphate kinase, which is a polypeptide having an ATP regeneration ability.

(4. Production method)

In an embodiment of the present invention, provided is a method for producing γ-Glu-X-Gly where X represents an amino acid, the method including causing a transformant expressing the above-described polypeptide in accordance with an embodiment of the present invention or the above-described polynucleotide in accordance with an embodiment of the present invention and/or a treated material of the transformant to act on γ-glutamyl dipeptide.

By causing a transformant expressing a polypeptide in accordance with an embodiment of the present invention or a polynucleotide in accordance with an embodiment of the present invention and/or a treated material of the transformant to act on γ-glutamyl dipeptide, more preferably γ-glutamyl cysteine, oxidized γ-glutamyl cysteine, or γ-glutamyl alanine, it is possible to produce γ-Glu-X-Gly (where X represents an amino acid), more preferably reduced glutathione, oxidized glutathione, or γ-glutamyl-alanyl-glycine, to each of which amino acids equivalent to a single molecule are further added.

A peptide to be used as a substrate of a transformant expressing a polypeptide in accordance with an embodiment of the present invention or a polynucleotide in accordance with an embodiment of the present invention and/or a treated material of the transformant is not particularly limited. However, in a case where a reaction of adding glycine to γ-glutamyl cysteine and oxidized γ-glutamyl cysteine is carried out, a product obtained from the reaction is glutathione, which is useful. Such a reaction is therefore a very beneficial reaction.

In a case where a peptide extension reaction is carried out with use of the above peptide as a substrate as well as a transformant expressing a polypeptide in accordance with an embodiment of the present invention or a polypeptide in accordance with an embodiment of the present invention and/or a treated material of the transformant, a method by which the peptide extension reaction is carried out can be, but not limited to, the following method.

Specifically, an appropriate solvent (e.g., 50 mM Tris-HCl buffer solution (pH: 8.0) or the like) and a peptide serving as a substrate (e.g., γ-glutamyl cysteine, oxidized γ-glutamyl cysteine, or γ-glutamyl alanine) are added, and a coenzyme such as magnesium sulfate or ATP and a culture of a transformant in accordance with an embodiment of the present invention and/or a treated material of the culture are added. This mixture is reacted with stirring at a controlled pH. At this time, apart from the transformant expressing a polypeptide in accordance with an embodiment of the present invention, a transformant expressing the above-described polypeptide having an ATP regeneration ability and a culture of the transformant, and/or a treated material of the transformant and the culture, and the like may be added.

As used herein, "a treated material of a transformant" means, for example, a cell-free extract, a crude extract, cultured bacterial cells, a freeze-dried organism, an acetone-dried organism, disrupted bacterial cells, a mixture or fixed material of these, or the like in which a polypeptide enzyme catalytic activity of a polypeptide in accordance with an embodiment of the present invention remains. A polypeptide in accordance with an embodiment of the present invention itself, a fixed material of a transformant expressing a polynucleotide in accordance with an embodiment of the present invention, and the like are also encompassed in the scope of the "treated material of a transformant."

A reaction between (i) a transformant expressing a polypeptide in accordance with an embodiment of the present invention or a polynucleotide in accordance with an embodiment of the present invention and/or a treated material of the transformant and (ii) a substrate is carried out at a temperature of 5°C to 80°C, preferably 10°C to 70°C, more preferably 20°C to 70°C. A pH of the reaction solution during the reaction is maintained at 3 to 10, preferably 4 to 10, more preferably 5 to 9. The reaction may be carried out in batches or continuously. In a case where the reaction is carried out in batches, the substrate for the reaction may be added so that a concentration of the substrate is 0.01% to 100% (w/v), preferably 0.1% to 70%, more preferably 0.5% to 50%. Further, an additional amount of the substrate may be newly added during the reaction.

A reaction between (i) a transformant expressing a polypeptide in accordance with an embodiment of the present invention or a polynucleotide in accordance with an embodiment of the present invention and/or a treated material of the transformant and (ii) a substrate may be carried out with use of an aqueous solvent or a mixture of an aqueous solvent and an organic solvent. Examples of the organic solvent encompass dimethylformamide, dimethyl sulfoxide, 2-propanol, ethyl acetate, toluene, methanol, ethanol, n-butanol, hexane, acetonitrile, propyl acetate, butyl acetate, acetone, dimethoxypropane, t-methyl butyl ether, diethyl ether, diisopropyl ether, dioxane, tetrahydrofuran, dimethylacetamide, diglyme, ethylene glycol, dimethoxyethane, carbon tetrachloride, methylene chloride, ethyl cellosolve, cellosolve acetate, 1,3-dimethyl-2-imidazolidinone, hexamethylphosphoric triamide, and the like.

In a case where a reaction between (i) a transformant expressing a polypeptide in accordance with an embodiment of the present invention or a polynucleotide in accordance with an embodiment of the present invention and/or a treated material of the transformant and (ii) a substrate is carried out, a significant reduction in amount of the coenzyme ATP used can be achieved by using a transformant capable of producing both a polypeptide in accordance with an embodiment of the present invention and a polypeptide having an ATP regeneration ability or by separately adding a transformant capable of producing a polypeptide having an ATP regeneration ability. The following description will discuss in detail a polypeptide having an ATP regeneration ability.

In a case where a reaction of synthesizing γ-Glu-X-Gly, reduced glutathione, and/or oxidized glutathione with use of a transformant capable of producing a polypeptide in accordance with an embodiment of the present invention, ATP is required as a coenzyme. As described above, the reaction can be carried out by adding a necessary amount of ATP to the reaction system. It is possible, however, to achieve a significant reduction in amount of expensive ATP by carrying out the reaction by using, together with the substrate, an enzyme having an ability (hereinafter referred to as "ATP regeneration ability" to convert the coenzyme (ADP or AMP) which has been dephosphorylated into ATP, that is, by using an ATP regenerating system in combination with a polypeptide in accordance with an embodiment of the present invention. As the enzyme having an ATP regeneration ability, polyphosphate kinase, adenylate kinase, pyruvate kinase, acetate kinase, phosphocreatine kinase, and the like may be used alone or in combination of two or more thereof. Preferably, polyphosphate kinase and/or adenylate kinase is/are used.

The reaction with use of the ATP regenerating system can be carried out by adding the ATP regenerating system into a γ-Glu-X-Gly synthesis reaction system, a reduced glutathione synthesis reaction system, or an oxidized glutathione synthesize reaction system. However, in a case where a transformant which has been transformed with use of both a polynucleotide encoding a polypeptide in accordance with an embodiment of the present invention and a polynucleotide encoding a polypeptide having an ATP regeneration ability is used as a catalyst, the reaction can be efficiently carried out without separately preparing an enzyme having an ATP regeneration ability and adding the enzyme into the reaction system. Such a transformant can be obtained in accordance with the above-described method.

A method for collecting a product from the reaction solution after the synthesis reaction is not particularly limited, but the product can be obtained easily (i) by directly purifying the product from the reaction solution or (ii) by separating bacterial cells and the like from the reaction, then extracting the product with use of a solvent such as methanol, dehydating the product, and then purifying the product by distillation, recrystallization, silica gel column chromatography, column chromatography with use of a synthetic adsorbent, or the like.

## Examples

The following description will detail the present invention in Examples. Note, however, that the present invention is not limited to these Examples. Note that a detailed operation method and others related to a recombinant DNA technology used in Examples below are described in the following literature:
Molecular Cloning 2nd Edition (Joseph Sambrook, Cold Spring Harbor Laboratory Press (1989)), Current Protocols in Molecular Biology (Frederick M. Ausubel, Greene Publishing Associates and Wiley-Interscience (1989))

### (Reference Example 1) Construction of recombinant vector PTDGSH2

A gene sequence (SEQ ID NO: 2), in which a gene encoding *Thiobacillus denifitricans* ATCC25259-derived glutathione synthetase (NCBI Reference Sequence: WP_011312921) was subjected to codon optimization so as to be adapted to an *Escherichia coli* host, was chemically synthesized by Eurofins Genomics K.K. to have an NdeI site added to the 5' end and an EcoRI site added to the 3'end. The gene thus obtained was digested with NdeI and EcoRI and inserted between an NdeI recognition site and an EcoRI recognition site downstream of a lac promoter of a plasmid pUCN18 (a plasmid obtained by modifying T at position 185 of pUC18 (manufactured by Takara-Bio Inc.) to A by means of PCR so as to destroy an NdeI site and further modifying GC at positions 471-472 to TG so as to newly introduce an NdeI site) to construct a recombinant vector pTDGSH2.

### (Reference Example 2) Preparation of recombinant organisms expressing polypeptide

With use of the recombinant vector pTDGSH2 constructed in Reference Example 1, an *E. coli* HB101 competent cell (manufactured by Takara-Bio Inc.) was transformed to obtain a recombinant organism *E. coli* HB101 (pTDGSH2). In addition, with use of the pUCN18, an *E. coli* HB101 competent cell (manufactured by Takara-Bio Inc.) was transformed to obtain a recombinant organism *E. coli* HB101 (pUCN18).

### (Reference Example 3) Expression of DNA in recombinant organisms

Two types of recombinant organisms (*E. coli* HB101 (pUCN18) and *E. coli* HB101 (pTDGSH2)) obtained in Reference Example 2 were each inoculated onto 5 ml of 2 × YT medium (tryptone: 1.6%, yeast extract: 1.0%, sodium chloride: 0.5%, pH: 7.0) containing 200 μg/ml of ampicillin, and were each subjected to shake culture at 37°C for 24 hours. Each culture solution thus obtained by the above culture was subjected to centrifugal separation to collect bacterial cells, and the bacterial cells were then suspended in 1ml of a 200 mM Tris-HCl buffer solution (pH: 8.5). Resultant suspensions were each disrupted by means of a UH-50 ultrasonic homogenizer (manufactured by SMT Co., Ltd.), and were then subjected to centrifugation so as to remove bacterial cell debris. Thus obtained were cell-free extracts. Glutathione synthetase activity of each of these cell-free extracts was measured. Glutathione synthetase activity was quantified through HPLC analysis of oxidized glutathione produced by adding 15 mM oxidized γ-glutamyl cysteine, 30 mM glycine, 30 mM ATP, 10 mM magnesium sulfate, and each of the cell-free extracts to 200 mM Tris-HCl buffer solution (pH: 8.5) and then carrying out reaction at 30°C for 10 minutes. In this reaction condition, enzyme activity of producing 1 μmol of oxidized glutathione for 1 minute was defined as 2U. Glutathione synthetase activities of the respective recombinant organisms are shown below.

For the *E. coli* HB101 (pUCN18), glutathione synthetase activity was not more than 0.1 mU/mg. Meanwhile, for the *E. coli* HB101 (pTDGSH2) which expressed TDGSH2, glutathione synthetic activity was 700 mU/mg. As described above, the recombinant organisms obtained in Reference Example 2 were confirmed to have glutathione synthetic activity and produce TDGSH2.

(Reference Example 4) Enzyme activity measurement with use of alternative substrate

Two types of recombinant organisms (*E. coli* HB101 (pUCN18) and *E. coli* HB101 (pTDGSH2)) obtained in Reference Example 2 were each inoculated onto 5 ml of 2 × YT medium (tryptone: 1.6%, yeast extract: 1.0%, sodium chloride: 0.5%, pH: 7.0) containing 200 μg/ml of ampicillin, and were each subjected to shake culture at 37°C for 24 hours. Each culture solution thus obtained by the above culture was subjected to centrifugal separation to collect bacterial cells, and the bacterial cells were then suspended in 1ml of a 200 mM Tris-HCl buffer solution (pH: 8.5). Resultant suspensions were each disrupted by means of a UH-50 ultrasonic homogenizer (manufactured by SMT Co., Ltd.), and were then subjected to centrifugation so as to remove bacterial cell debris. Thus obtained were cell-free extracts. These cell-free extracts were subjected to measurement of γ-glutamyl-alanyl-glycine synthetase activity. Quantification of γ-glutamyl-alanyl-glycine synthetase activity was performed through HPLC analysis of γ-glutamyl-alanyl-glycine produced by adding 20 mM γ-glutamyl alanine, 20 mM glycine, 20 mM ATP, 10 mM magnesium sulfate, and a 20-fold diluted solution obtained by diluting each of the cell-free extracts with a 200 mM Tris-HCl buffer solution (pH: 8.5) to a 200 mM Tris-HCl buffer solution (pH: 8.5), and then carrying out reaction at 30°C for 10 minutes. In this reaction condition, enzyme activity of producing 1 pmol of γ-glutamyl-alanyl-glycine for 1 minute was defined as 1U. γ-glutamyl-alanyl-glycine synthetase activities of the respective recombinant organisms are shown below.

For the *E. coli* HB101 (pUCN18), γ-glutamyl-alanyl-glycine synthetase activity was not more than 0.1 U/mg. Meanwhile, for the *E. coli* HB101 (pTDGSH2) which expressed TDGSH2, γ-glutamyl-alanyl-glycine synthetic activity was 6 U/mg. As described above, wild-type glutathione synthetase was confirmed to have γ-glutamyl-alanyl-glycine synthetic activity as well.

(Reference Example 5) Thermal stability of wild-type enzyme

Cell-free extracts of the wild-type enzyme were obtained in the same manner as in Reference Example 3. The cell-free extracts were each incubated at 60°C for 10 minutes, at 60°C for 30 minutes, at 70°C for 10 minutes, or at 70°C for 15 minutes. After the incubation, the resultant cell-free extracts were each diluted. A cell-free extract which had not been heated was diluted similarly. Glutathione synthetic activity of each of these cell-free extracts was measured in the same manner as in Reference Example 3. The remaining activity after heating was calculated in accordance with a formula below, and a calculated value of the remaining activity was used as an index of thermal stability. The results are shown in Table 1. Remaining activity (%) = [an enzyme activity after heating] ÷ [an enzyme activity before heating] × 100

[Table 1]

| 60°C | | 70°C | |
|---|---|---|---|
| 10 min. | 30 min. | 10 min. | 15 min. |
| 0 | 0 | 0 | 0 |

The wild-type enzymes, when heated under these conditions, were deactivated. Thus, their activities could not be detected.

(Example 1) Preparation 1 of mutated enzyme gene library

By using the plasmid pTDGSH2 containing the T. *denitrificans* derived glutathione synthetase gene prepared in Reference Example 1 as a template, a primer 1 (5'-GGGTTTCATATGAAACTGCTGTTCGTCG-3 ' (SEQ ID NO: 4 shown in the sequence listing)), and a primer 2 (5'-CCGGAATTCTTATCATTCCGGACGCG-3' (SEQ ID NO: 5 shown in the sequence listing)), DNA amplified fragments each having random mutations introduced over a full length of a RKP gene were obtained by error-prone PCR (Leung et al., Technique 1, 11-15 (1989)). The amplified fragments were each digested with restriction enzymes NdeI and EcoRI. After that, the amplified fragments were each integrated into a high expression vector pUCN18 treated with the same enzymes to prepare a plurality of mutant enzyme expressing plasmids. The plasmids thus prepared were each used to transform the *E. coli* HB101, and resultant transformants were spread on an LB plate medium containing 100 μg/mL of ampicillin. A grown colony is a colony of recombinant *Escherichia coli* having a mutation-introduced glutathione synthetase gene. Such a group of recombinant bacteria was defined as a mutated enzyme gene library 1.

(Example 2) Selection 1 of modified glutathione synthetase

From the mutated enzyme gene library 1, a modified glutathione synthetase having a higher thermal stability as compared with a wild-type glutathione synthetase was selected. The recombinant bacteria of the mutated enzyme gene library 1 prepared in Example 1 and the *E. coli* HB101 (pTDGSH2) (control) prepared in Reference Example 2 were each cultured in the same manner as in Reference Example 3. Each culture solution thus obtained was subjected to centrifugal separation to remove a supernatant, and was suspended in, for example, a 0.2 M Tris-HCl buffer solution (pH: 8.5). This suspension was disrupted, and then subjected to centrifugation so as to remove a precipitate. Thus obtained were cell-free extracts. The cell-free extracts containing respective enzymes were each heated at 60°C. After 30 minutes of heating, each cell-free extract was dispensed onto a 96-well plate (manufactured by AGC TECHNO GLASS CO., LTD.), and a 0.2 M Tris-HCl buffer solution (pH: 8.5) containing 30 mM ATP disodium salt, 10 mM magnesium sulfate heptahydrate, 15 mM oxidized γ-glutamyl cysteine, and 30 mM glycine was added so as to carry out incubation at 30°C for 3 hours. The reaction solution was dispensed onto another 96-well plate (manufactured by AGC TECHNO GLASS CO., LTD.), and a 50 mM Tris-HCl buffer solution (pH: 8.0) containing glutathione reductase (30 unit/L, manufactured by Sigma-Aldrich) and 1.2 mM NADPH was added so as to carry out incubation at room temperature for 2 minutes. In this process, oxidized glutathione produced by glutathione synthetase is converted into reduced glutathione. To this, a 50 mM Tris-HCl buffer solution (pH: 8.0) containing 0.2 mg/mL of DTNB was added, and detection of absorption of light at 405 nm was carried out over time. A ratio of light absorption of a sample obtained by a glutathione synthesis reaction with use of a cell-free extract that had been subjected to a heat treatment to light absorption of a control obtained by a glutathione synthesis reaction with use of a cell-free extract that had not been subjected to a heat treatment was defined as an activity residual rate. A sample whose activity residual rate was higher than that of wild-type glutathione synthetase was selected as an enzyme having an improved thermal stability. Plasmids were extracted from the culture solution of the enzyme thus selected, and with use of Big Dye Terminator Cycle Sequencing Kit (manufactured by Applied Biosystems Japan, Ltd.) and Applied Biosystems 3130xl Genetic Analyzer (manufactured by Applied Biosystems Japan, Ltd.), the base sequence of a modified glutathione synthetase gene was determined, so that a mutation site(s) was identified. Table 2 shows the mutation site(s) of the obtained modified glutathione synthetase having an improved thermal stability.

[Table 2]

| Name of plasmid | Mutation site |
|---|---|
| pTDGSH2m01 | L13S |
| pTDGSH2m02 | K17E/R113H/T230P |
| pTDGSH2m03 | S20T/E215D |
| pTDGSH2m04 | S20T/R241H |
| pTDGSH2m05 | M23L/I126N |
| pTDGSH2m06 | F78L/T278A |
| pTDGSH2m07 | G101S |
| pTDGSH2m08 | A125V/D249E |
| pTDGSH2m09 | A125V/H152Q |
| pTDGSH2m10 | P136T |
| pTDGSH2m11 | V138A/L149Q/R241H |
| pTDGSH2m12 | D154N/S246R |
| pTDGSH2m13 | I155L/T239S |
| pTDGSH2m14 | R197Q |

(continued)

| Name of plasmid | Mutation site |
|---|---|
| pTDGSH2m15 | V260A |
| pTDGSH2m16 | S262C |
| pTDGSH2m17 | L270I |

17 types of enzymes having an improved thermal stability shown in Table 2 were obtained.

(Example 3) Evaluation 1 of modified glutathione synthetase

The recombinant bacteria of the modified glutathione synthetase obtained in Example 2 and the *E. coli* HB101 (pTDGSH2) (control) prepared in Reference Example 2 were each cultured in the same manner as in Reference Examples 3 and 4. Each culture solution thus obtained was subjected to centrifugal separation to collect bacterial cells, and the bacterial cells were then suspended in a 0.2 M Tris-HCl buffer solution (pH: 8.5) in an amount equivalent to the amount of the culture solution. Resultant suspensions were each disrupted by means of a UH-50 ultrasonic homogenizer (manufactured by SMT Co., Ltd.), and were then subjected to centrifugation so as to remove bacterial cell debris. Thus obtained were cell-free extracts. These cell-free extracts were confirmed in the same manner as in Reference Examples 3 and 4 to have both glutathione synthetic activity and γ-glutamyl-alanyl-glycine synthetic activity. Table 3 shows a relative activity of modified glutathione synthetase in a case where glutathione synthetic activity of the wild-type enzyme is 100.

[Table 3]

| Mutation site | Relative activity (%) |
|---|---|
| Wild-type | 100 |
| S20T/E215D | 119 |
| S20T/R241H | 105 |
| G101S | 97 |
| A125V/D249E | 105 |
| A125V/H152Q | 102 |
| P136T | 77 |
| D154N/S246R | 70 |
| I155L/T239S | 111 |
| R197Q | 106 |
| V260A | 152 |
| S262C | 121 |
| L270I | 106 |

(Example 4) Evaluation 2 of modified glutathione synthetase

The recombinant bacteria of the modified glutathione synthetase obtained in Example 2 and the *E. coli* HB101 (pTDGSH2) (control) prepared in Reference Example 3 were each cultured in the same manner as in Reference Examples 3 and 4. Each culture solution thus obtained was subjected to centrifugal separation to collect bacterial cells, and the bacterial cells were then suspended in a 0.2 M Tris-HCl buffer solution (pH: 8.5) in an amount equivalent to the amount of the culture solution. Resultant suspensions were each disrupted by means of a UH-50 ultrasonic homogenizer (manufactured by SMT Co., Ltd.), and were then subjected to centrifugation so as to remove bacterial cell debris. Thus obtained were cell-free extracts. The cell-free extracts were each heated at 60°C for 10 minutes. With use of diluted solutions of the heated cell-free extracts and a diluted solution of a non-heated cell-free extract, γ-glutamyl-alanyl-glycine synthetic activity was measured by the method described in Reference Example 4. The remaining activity after heating was calculated in accordance with a formula below, and a calculated value of the remaining activity was used as an index of thermal stability.

$$\text{Remaining activity (\%)} = [\text{an enzyme activity after heating}] \div [\text{an enzyme activity before heating}] \times 100$$

Table 4 shows relative activities between the wild-type enzyme and the modified glutathione synthetases both of which were obtained through heating at 60°C for 10 minutes and were then evaluated.

[Table 4]

| Mutation site | Remaining activity (%) |
|---|---|
| Wild-type | 0 |
| S20T/E215D | 71 |
| S20T/R241H | 60 |
| G101S | 66 |
| A125V/D249E | 50 |
| V138A/L149Q/R241H | 25 |
| I155L/T239S | 27 |
| R197Q | 6 |
| V260A | 74 |
| S262C | 14 |
| L270I | 12 |

The modified glutathione synthetases shown in Table 4 had thermal stability higher than that of the wild-type enzyme.

(Example 5) Evaluation 3 of modified glutathione synthetase

The recombinant bacteria of the modified glutathione synthetase obtained in Example 2 and the *E. coli* HB101 (pTDGSH2) (control) prepared in Reference Example 3 were each cultured in the same manner as in Reference Examples 3 and 4. Each culture solution thus obtained was subjected to centrifugal separation to collect bacterial cells, and the bacterial cells were then suspended in a 0.2 M Tris-HCl buffer solution (pH: 8.5) in an amount equivalent to the amount of the culture solution. Resultant suspensions were each disrupted by means of a UH-50 ultrasonic homogenizer (manufactured by SMT Co., Ltd.), and were then subjected to centrifugation so as to remove bacterial cell debris. Thus obtained were cell-free extracts. The cell-free extracts were each heated at 60°C for 30 minutes. With use of diluted solutions of the heated cell-free extracts and a diluted solution of a non-heated cell-free extract, γ-glutamyl-alanyl-glycine synthetic activity was measured by the method described in Reference Example 4. The remaining activity after heating was calculated in accordance with a formula below, and a calculated value of the remaining activity was used as an index of thermal stability.

$$\text{Remaining activity (\%)} = [\text{an enzyme activity after heating}] \div [\text{an enzyme activity before heating}] \times 100$$

Table 5 shows relative activities between the wild-type enzyme and the modified glutathione synthetases both of which were obtained through heating at 60°C for 30 minutes and were then evaluated.

[Table 5]

| Mutation site | Remaining activity (%) |
|---|---|
| Wild-type | 0 |
| L13S | 24 |
| K17E/R113H/T230P | 15 |
| M23L/I126N | 51 |
| F78L/T278A | 31 |

(continued)

| Mutation site | Remaining activity (%) |
|---|---|
| A125V/H152Q | 7 |
| P136T | 15 |
| D154N/S246R | 16 |

The modified glutathione synthetases shown in Table 5 had thermal stability higher than that of the wild-type enzyme.

(Example 6) Evaluation 4 of modified glutathione synthetase

The recombinant bacteria of the modified glutathione synthetase obtained in Example 2 and the *E. coli* HB101 (pTDGSH2) (control) prepared in Reference Example 3 were each cultured in the same manner as in Reference Examples 3 and 4. Each culture solution thus obtained was subjected to centrifugal separation to collect bacterial cells, and the bacterial cells were then suspended in a 0.2 M Tris-HCl buffer solution (pH: 8.5) in an amount equivalent to the amount of the culture solution. Resultant suspensions were each disrupted by means of a UH-50 ultrasonic homogenizer (manufactured by SMT Co., Ltd.), and were then subjected to centrifugation so as to remove bacterial cell debris. Thus obtained were cell-free extracts. The cell-free extracts were each heated at 70°C for 15 minutes. With use of diluted solutions of the heated cell-free extracts and a diluted solution of a non-heated cell-free extract, γ-glutamyl-alanyl-glycine synthetic activity was measured by the method described in Reference Example 4. The remaining activity after heating was calculated in accordance with a formula below, and a calculated value of the remaining activity was used as an index of thermal stability.

$$\text{Remaining activity (\%)} = [\text{an enzyme activity after heating}] \div [\text{an enzyme activity before heating}] \times 100$$

Table 6 shows relative activities between the wild-type enzyme and the modified glutathione synthetases both of which were obtained through heating at 70°C for 15 minutes and were then evaluated.

[Table 6]

| Mutation site | Remaining activity (%) |
|---|---|
| Wild-type | 0 |
| L13S | 22 |
| K17E/R113H/T230P | 14 |
| M23L/I126N | 52 |
| F78L/T278A | 28 |
| A125V/H152Q | 1 |
| P136T | 2 |
| D154N/S246R | 2 |

The modified glutathione synthetases shown in Table 6 had thermal stability higher than that of the wild-type enzyme.

(Example 7) Preparation 2 of mutated enzyme gene library

By using the plasmid pTDGSH2m15 (see Table 2) obtained in Example 2 as a template, a primer 1 (5'-GGGTTTCATATGAAACTGCTGTTCGTCG-3' (SEQ ID NO: 4 shown in the sequence listing)), and a primer 2 (5'-CCGGAATTCTTATCATTCCGGACGCG-3' (SEQ ID NO: 5 shown in the sequence listing)), DNA amplified fragments each having random mutations introduced over a full length of a glutathione synthetase gene were obtained by error-prone PCR (Leung et al., Technique 1, 11-15 (1989)). The amplified fragments were each digested with restriction enzymes NdeI and EcoRI. After that, the amplified fragments were each integrated into a high expression vector pUCN18 treated with the same enzymes to prepare a plurality of mutant enzyme expressing plasmids. The plasmids thus prepared were each used to transform the *E. coli* HB101, and resultant transformants were spread on an LB plate medium containing 100

μg/mL of ampicillin. A grown colony is a colony of recombinant *Escherichia coli* having a mutation-introduced glutathione synthetase gene. Such a group of recombinant bacteria was defined as a mutated enzyme gene library 2.

(Example 8) Selection 2 of modified glutathione synthetase

From the mutated enzyme gene library 2, a modified glutathione synthetase having a higher thermal stability as compared with a wild-type glutathione synthetase was selected. The recombinant bacteria of the mutated enzyme gene library 2 prepared in Example 7, the *E. coli* HB101 (pTDGSH2) (control) prepared in Reference Example 2, and the *E.coli* HB101 (pTDGSH2m15) obtained in Example 2 were each cultured in the same manner as in Reference Example 3. Each culture solution thus obtained was subjected to centrifugal separation to remove a supernatant, and was suspended in, for example, a 0.2 M Tris-HCl buffer solution (pH: 8.5). This suspension was disrupted, and then subjected to centrifugation so as to remove a precipitate. Thus obtained were cell-free extracts. The cell-free extracts containing respective enzymes were each heated at 60°C. After 40 minutes of heating, each cell-free extract was dispensed onto a 96-well plate (manufactured by AGC TECHNO GLASS CO., LTD.), and a 0.2 M Tris-HCl buffer solution (pH: 8.5) containing 30 mM ATP disodium salt, 10 mM magnesium sulfate heptahydrate, 15 mM oxidized γ-glutamyl cysteine, and 30 mM glycine was added so as to carry out incubation at 30°C for 3 hours. The reaction solution was dispensed onto another 96-well plate (manufactured by AGC TECHNO GLASS CO., LTD.), and a 50 mM Tris-HCl buffer solution (pH: 8.0) containing glutathione reductase (30 unit/L, manufactured by Sigma-Aldrich) and 1.2 mM NADPH was added so as to carry out incubation at room temperature for 2 minutes. In this process, oxidized glutathione produced by glutathione synthetase is converted into reduced glutathione. To this, a 50 mM Tris-HCl buffer solution (pH: 8.0) containing 0.2 mg/mL of DTNB was added, and detection of absorption of light at 405 nm was carried out over time. A ratio of light absorption of a sample obtained by a glutathione synthesis reaction with use of a cell-free extract that had been subjected to a heat treatment to light absorption of a control obtained by a glutathione synthesis reaction with use of a cell-free extract that had not been subjected to a heat treatment was defined as an activity residual rate. A sample whose activity residual rate was higher than that of wild-type glutathione synthetase and modified enzyme produced by the *E. coli* HB101 (pTDGSH2m15) was selected as an enzyme having an improved thermal stability. Plasmids were extracted from the culture solution of the enzyme thus selected, and with use of Big Dye Terminator Cycle Sequencing Kit (manufactured by Applied Biosystems Japan, Ltd.) and Applied Biosystems 3130xl Genetic Analyzer (manufactured by Applied Biosystems Japan, Ltd.), the base sequence of a modified glutathione synthetase gene was determined, so that a mutation site(s) was identified. Table 7 shows the mutation site(s) of the obtained modified glutathione synthetase having an improved thermal stability.

[Table 7]

| Name of plasmid | Mutation site |
|---|---|
| pTDGSH2m18 | A39T/V260A |
| pTDGSH2m19 | T70S/V260A |
| pTDGSH2m20 | G101S/V260A |
| pTDGSH2m21 | P200S/V260A |
| pTDGSH2m22 | L226R/V260A |
| pTDGSH2m23 | T227S/V260A |
| pTDGSH2m24 | E254D/V260A |
| pTDGSH2m25 | V260A/D278G/I1307V |
| pTDGSH2m26 | V260A/V299A |
| pTDGSH2m27 | V260A/A305G |
| pTDGSH2m28 | V260A/A310T |

11 types of enzymes having an improved thermal stability shown in Table 7 were obtained.

(Example 9) Evaluation 5 of modified glutathione synthetase

The recombinant bacteria of the modified glutathione synthetase obtained in Example 8 and the *E. coli* HB101 (pTDGSH2) (control) prepared in Reference Example 2 were each cultured in the same manner as in Reference Examples 3 and 4. Each culture solution thus obtained was subjected to centrifugal separation to collect bacterial cells, and the bacterial cells were then suspended in a 0.2 M Tris-HCl buffer solution (pH: 8.5) in an amount equivalent to the amount of the culture solution. Resultant suspensions were each disrupted by means of a UH-50 ultrasonic homogenizer (manufactured by SMT Co., Ltd.), and were then subjected to centrifugation so as to remove bacterial cell debris. Thus obtained were cell-free extracts. These cell-free extracts were confirmed in the same manner as in Reference Examples 3 and 4 to have both glutathione synthetic activity and γ-glutamyl-alanyl-glycine synthetic activity. Table 8 shows a relative activity of modified glutathione synthetase in a case where glutathione synthetic activity of the wild-type enzyme is 100.

[Table 8]

| Mutation site | Relative activity (%) |
|---|---|
| Wild-type | 100 |
| A39T/V260A | 129 |
| T70S/V260A | 123 |
| G101S/V260A | 137 |
| P200S/V260A | 114 |
| L226R/V260A | 121 |
| T227S/V260A | 132 |
| E254D/V260A | 116 |
| V260A/D278G/I307V | 101 |
| V260A/V299A | 105 |
| V260A/A305G | 123 |
| V260A/A310T | 123 |

(Example 10) Evaluation 6 of modified glutathione synthetase

The recombinant bacteria of the modified glutathione synthetase obtained in Example 8 and the *E. coli* HB101 (pTDGSH2) (control) prepared in Reference Example 3 were each cultured in the same manner as in Reference Examples 3 and 4. Each culture solution thus obtained was subjected to centrifugal separation to collect bacterial cells, and the bacterial cells were then suspended in a 0.2 M Tris-HCl buffer solution (pH: 8.5) in an amount equivalent to the amount of the culture solution. Resultant suspensions were each disrupted by means of a UH-50 ultrasonic homogenizer (manufactured by SMT Co., Ltd.), and were then subjected to centrifugation so as to remove bacterial cell debris. Thus obtained were cell-free extracts. The cell-free extracts were each heated at 60°C for 10 minutes. With use of diluted solutions of the heated cell-free extracts and a diluted solution of a non-heated cell-free extract, γ-glutamyl-alanyl-glycine synthetic activity was measured by the method described in Reference Example 4. The remaining activity after heating was calculated in accordance with a formula below, and a calculated value of the remaining activity was used as an index of thermal stability.

$$\text{Remaining activity (\%)} = [\text{an enzyme activity after heating}] \div [\text{an enzyme activity before heating}] \times 100$$

Table 9 shows relative activities between the wild-type enzyme and the modified glutathione synthetases both of which were obtained through heating at 60°C for 10 minutes and were then evaluated.

[Table 9]

| Mutation site | Remaining activity (%) |
|---|---|
| Wild-type | 0 |
| A39T/V260A | 86 |
| T70S/V260A | 76 |
| G101S/V260A | 91 |
| P200S/V260A | 84 |
| L226R/V260A | 83 |
| T227S/V260A | 90 |
| E254D/V260A | 80 |

(continued)

| Mutation site | Remaining activity (%) |
|---|---|
| V260A/D278G/I307V | 91 |
| V260A/V299A | 88 |
| V260A/A305G | 78 |
| V260A/A310T | 79 |

The modified glutathione synthetases shown in Table 9 had thermal stability higher than that of the wild-type enzyme.

(Example 11) Evaluation 7 of modified glutathione synthetase

The recombinant bacteria of the modified glutathione synthetase obtained in Example 8, the *E. coli* HB101 (pTDGSH2) (control) prepared in Reference Example 3, and the *E. coli* HB101 (pTDGSH2m15) obtained in Example 2 were each cultured in the same manner as in Reference Examples 3 and 4. Each culture solution thus obtained was subjected to centrifugal separation to collect bacterial cells, and the bacterial cells were then suspended in a 0.2 M Tris-HCl buffer solution (pH: 8.5) in an amount equivalent to the amount of the culture solution. Resultant suspensions were each disrupted by means of a UH-50 ultrasonic homogenizer (manufactured by SMT Co., Ltd.), and were then subjected to centrifugation so as to remove bacterial cell debris. Thus obtained were cell-free extracts. The cell-free extracts were each heated at 70°C for 10 minutes. With use of diluted solutions of the heated cell-free extracts and a diluted solution of a non-heated cell-free extract, γ-glutamyl-alanyl-glycine synthetic activity was measured by the method described in Reference Example 4. The remaining activity after heating was calculated in accordance with a formula below, and a calculated value of the remaining activity was used as an index of thermal stability.

$$\text{Remaining activity (\%)} = [\text{an enzyme activity after heating}] \div [\text{an enzyme activity before heating}] \times 100$$

Table 10 shows remaining activities of the wild-type enzyme and the modified glutathione synthetases both of which were obtained through heating at 70°C for 10 minutes and were then evaluated.

[Table 10]

| Mutation site | Remaining activity (%) |
|---|---|
| Wild-type | 0 |
| V260A | 40 |
| A39T/V260A | 75 |
| T70S/V260A | 19 |
| G101S/V260A | 89 |
| P200S/V260A | 34 |
| T227S/V260A | 38 |
| E254D/V260A | 42 |
| V260A/D278G/I307V | 46 |
| V260A/A305G | 30 |
| V260A/A310T | 49 |

The modified glutathione synthetases shown in Table 10 had thermal stability higher than that of the wild-type enzyme. Further, it was shown that addition of another mutation to V260A mutation further improves thermal stability.

(Example 12) Preparation 1 of modified glutathione synthetase having substitution at position 260

By using the plasmid pTDGSH2 prepared in Reference Example 3 as a template, a primer 3 (5'-ACGATTGCCCCTTGGTGTCGCAGCCAGGGCATT-3' (SEQ ID NO: 6 shown in the sequence listing)), and a primer 4 (5'-AATGCCCTGGCTGCGACACCAAGGGGCAATCGT-3' (SEQ ID NO: 7 shown in the sequence listing)), PCR was performed to amplify a full-length plasmid having amino acid substitution of V260C in an amino acid sequence represented by SEQ ID NO: 1. 100 μL of a resultant PCR reaction solution was digested with DpnI. With use of a resultant reaction solution, an *E. coli* (HB101) competent cell (manufactured by Takara-Bio Inc.) was transformed to obtain a recombinant organism *E. coli* HB101 (pTDGSH2m29) which produces a modified glutathione synthetase V260C.

(Example 13) Preparation 2 of modified glutathione synthetase having substitution at position 260

By using the plasmid pTDGSH2 prepared in Reference Example 3 as a template, a primer 5 (5'-ACGATTGCCCCTTGGGGTCGCAGCCAGGGCATT-3' (SEQ ID NO: 8 shown in the sequence listing)), and a primer 6 (5'-AATGCCCTGGCTGCGACCCCAAGGGGCAATCGT-3' (SEQ ID NO: 9 shown in the sequence listing)), PCR was performed to amplify a full-length plasmid having amino acid substitution of V260G in an amino acid sequence represented by SEQ ID NO: 1. 100 μL of a resultant PCR reaction solution was digested with DpnI. With use of a resultant reaction solution, an *E. coli* (HB101) competent cell (manufactured by Takara-Bio Inc.) was transformed to obtain a recombinant organism *E. coli* HB101 (pTDGSH2m30) which produces a modified glutathione synthetase V260G.

(Example 14) Preparation 3 of modified glutathione synthetase having substitution at position 260

By using the plasmid pTDGSH2 prepared in Reference Example 3 as a template, a primer 7 (5'-ACGATTGCCCCTTGGCAGCGCAGCCAGGGCATT-3' (SEQ ID NO: 10 shown in the sequence listing)), and a primer 8 (5'-AATGCCCTGGCTGCGCTGCCAAGGGGCAATCGT-3' (SEQ ID NO: 11 shown in the sequence listing)), PCR was performed to amplify a full-length plasmid having amino acid substitution of V260Q in an amino acid sequence represented by SEQ ID NO: 1. 100 μL of a resultant PCR reaction solution was digested with DpnI. With use of a resultant reaction solution, an *E. coli* (HB101) competent cell (manufactured by Takara-Bio Inc.) was transformed to obtain a recombinant organism *E. coli* HB101 (pTDGSH2m31) which produces a modified glutathione synthetase V260Q.

(Example 15) Preparation 4 of modified glutathione synthetase having substitution at position 260

By using the plasmid pTDGSH2 prepared in Reference Example 3 as a template, a primer 9 (5'-ACGATTGCCCCTTGGACACGCAGCCAGGGCATT-3' (SEQ ID NO: 12 shown in the sequence listing)), and a primer 10 (5'-AATGCCCTGGCTGCGTGTCCAAGGGGCAATCGT-3' (SEQ ID NO: 13 shown in the sequence listing)), PCR was performed to amplify a full-length plasmid having amino acid substitution of V260T in an amino acid sequence represented by SEQ ID NO: 1. 100 μL of a resultant PCR reaction solution was digested with DpnI. With use of a resultant reaction solution, an *E. coli* (HB101) competent cell (manufactured by Takara-Bio Inc.) was transformed to obtain a recombinant organism *E. coli* HB101 (pTDGSH2m32) which produces a modified glutathione synthetase V260T.

(Example 16) Preparation 1 of modified glutathione synthetase having substitution at position 101

By using the plasmid pTDGSH2 prepared in Reference Example 3 as a template, a primer 11 (5'-GCGACGCACCTGTTAAACGTAGCCGAAACCAAC-3' (SEQ ID NO: 14 shown in the sequence listing)), and a primer 12 (5'-GTTGGTTTCGGCTACCTTTAACAGGTGCGTCGC-3' (SEQ ID NO: 15 shown in the sequence listing)), PCR was performed to amplify a full-length plasmid having amino acid substitution of G101N in an amino acid sequence represented by SEQ ID NO: 1. 100 μL of a resultant PCR reaction solution was digested with DpnI. With use of a resultant reaction solution, an *E. coli* (HB101) competent cell (manufactured by Takara-Bio Inc.) was transformed to obtain a recombinant organism *E. coli* HB101 (pTDGSH2m33) which produces a modified glutathione synthetase G101N.

(Example 17) Preparation 2 of modified glutathione synthetase having substitution at position 101

By using the plasmid pTDGSH2 prepared in Reference Example 3 as a template, a primer 13 (5'-GCGACGCACCTGTTACAGGTAGCCGAAACCAAC-3' (SEQ ID NO: 16 shown in the sequence listing)), and a primer 14 (5'-GTTGGTTTCGGCTACCTGTAACAGGTGCGTCGC-3' (SEQ ID NO: 17 shown in the sequence listing)), PCR was performed to amplify a full-length plasmid having amino acid substitution of G101Q in an amino acid sequence represented by SEQ ID NO: 1. 100 μL of a resultant PCR reaction solution was digested with DpnI. With use of a resultant reaction solution, an *E. coli* (HB101) competent cell (manufactured by Takara-Bio Inc.) was transformed to obtain a recombinant organism *E. coli* HB101 (pTDGSH2m34) which produces a modified glutathione synthetase G101Q.

(Example 18) Preparation 3 of modified glutathione synthetase having substitution at position 101

By using the plasmid pTDGSH2 prepared in Reference Example 3 as a template, a primer 15 (5'-GCGACGCACCTGTTAACCGTAGCCGAAACCAAC-3' (SEQ ID NO: 18 shown in the sequence listing)), and a primer 16 (5'-GTTGGTTTCGGCTACGGTTAACAGGTGCGTCGC-3' (SEQ ID NO: 19 shown in the sequence listing)), PCR was performed to amplify a full-length plasmid having amino acid substitution of G101T in an amino acid sequence represented by SEQ ID NO: 1. 100 μL of a resultant PCR reaction solution was digested with DpnI. With use of a resultant reaction solution, an *E. coli* (HB101) competent cell (manufactured by Takara-Bio Inc.) was transformed to obtain a recombinant organism *E. coli* HB101 (pTDGSH2m35) which produces a modified glutathione synthetase G101T.

(Example 19) Evaluation 8 of modified glutathione synthetase

The recombinant bacteria of the modified glutathione synthetases obtained in Examples 12 to 18 and the *E. coli* HB101 (pTDGSH2) (control) prepared in Reference Example 2 were each cultured in the same manner as in Reference Examples 3 and 4. Each culture solution thus obtained was subjected to centrifugal separation to collect bacterial cells, and the bacterial cells were then suspended in a 0.2 M Tris-HCl buffer solution (pH: 8.5) in an amount equivalent to the amount of the culture solution. Resultant suspensions were each disrupted by means of a UH-50 ultrasonic homogenizer (manufactured by SMT Co., Ltd.), and were then subjected to centrifugation so as to remove bacterial cell debris. Thus obtained were cell-free extracts. These cell-free extracts were confirmed in the same manner as in Reference Examples 3 and 4 to have both glutathione synthetic activity and γ-glutamyl-alanyl-glycine synthetic activity. Table 11 shows a relative activity of modified glutathione synthetase in a case where glutathione synthetic activity of the wild-type enzyme is 100.

[Table 11]

| Mutation site | Relative activity (%) |
|---|---|
| Wild-type | 100 |
| V260C | 345 |
| V260G | 70 |
| G101N | 95 |

(Example 20) Evaluation 9 of modified glutathione synthetase

The recombinant bacteria of the modified glutathione synthetases obtained in Examples 12 to 18 and the *E. coli* HB101 (pTDGSH2) (control) prepared in Reference Example 3 were each cultured in the same manner as in Reference Examples 3 and 4. Each culture solution thus obtained was subjected to centrifugal separation to collect bacterial cells, and the bacterial cells were then suspended in a 0.2 M Tris-HCl buffer solution (pH: 8.5) in an amount equivalent to the amount of the culture solution. Resultant suspensions were each disrupted by means of a UH-50 ultrasonic homogenizer (manufactured by SMT Co., Ltd.), and were then subjected to centrifugation so as to remove bacterial cell debris. Thus obtained were cell-free extracts. The cell-free extracts were each heated at 60°C for 10 minutes. With use of diluted solutions of the heated cell-free extracts and a diluted solution of a non-heated cell-free extract, γ-glutamyl-alanyl-glycine synthetic activity was measured by the method described in Reference Example 4. The remaining activity after heating was calculated in accordance with a formula below, and a calculated value of the remaining activity was used as an index of thermal stability.

$$\text{Remaining activity (\%)} = [\text{an enzyme activity after heating}] \div [\text{an enzyme activity before heating}] \times 100$$

Table 12 shows remaining activities of the wild-type enzyme and the modified glutathione synthetases both of which were obtained through heating at 60°C for 10 minutes and were then evaluated.

[Table 12]

| Mutation site | Remaining activity (%) |
|---|---|
| Wild-type | 0 |

(continued)

| Mutation site | Remaining activity (%) |
|---|---|
| V260C | 76 |
| V260G | 81 |
| V260Q | 47 |
| V260T | 41 |
| G101N | 49 |
| G101Q | 8 |
| G101T | 4 |

The modified glutathione synthetases shown in Table 12 had thermal stability higher than that of the wild-type enzyme.

(Reference Example 6) Preparation 6 of modified glutathione synthetase

The *E. coli* HB101 (pTDGSH2m15) obtained in Example 2 was inoculated onto 50 ml of 2YT medium (tryptone: 1.6%, yeast extract: 1.0%, NaCl: 0.5%, pH: 7.0) containing 200 μg/ml of ampicillin, and was subjected to shake culture at 37°C for 24 hours. Enzyme activity measured by the method described in (Reference Example 4) was 4 U/ml. Furthermore, adenylate kinase (ADK) activity derived from *E. coli* HB101, which had been used as host cells, measured by the method described international Publication No. 2016/002884 was 90 U/ml. Subsequently, the bacterial cells were collected by centrifugation, suspended in 2.5 ml of a 50 mM Tris-HCl buffer solution (pH: 8.0), and sonicated to obtain an enzyme solution.

(Example 21) Production 1 of glutathione with use of modified glutathione synthetase

Oxidized γ-glutamyl cysteine was synthesized by the method described in <Example 1> of International Publication No. 2016/002884. To a reaction solution obtained after 22 hours of this reaction were added 0.19 g (2.53 mmol) of glycine, 2 g of the modified glutathione synthetase (V260A) prepared in Reference Example 6, 2 g of PAP enzyme solution prepared by the same method as in Experiment 4 of International Publication No. 2016/002884. Then, reaction was started. At this time, pH was adjusted to 7.5 with 0.7 g of a 15 mass% aqueous sodium hydroxide solution. Analysis of a reaction solution obtained after 1 hour of reaction confirmed formation of oxidized glutathione. The yields were 6 mol% after 2.5 hours of reaction and 43 mol% after 14 hours of reaction, relative to starting L-cystine.

(Reference Example 7) Construction of expression vector for polyphosphate kinase

On the basis of the information described in International Publication No. 2006/080313, a gene sequence (SEQ ID NO: 20), in which a gene encoding a polypeptide in which N-terminus-side 82 amino acids of *Pseudomonas aeruginosa*-derived polyphosphate kinase (NCBI Reference Sequence: WP_023109529) were cleaved, and 83th asparagine was substituted with an initiation codon, methionine, was subjected to codon optimization so as to be adapted to an *Escherichia coli* host, was chemically synthesized by Eurofins Genomics K.K. to have an NdeI site added to the 5' end and an EcoRI site added to the 3'end. The gene thus obtained was digested with NdeI and EcoRI and inserted between an NdeI recognition site and an EcoRI recognition site downstream of a lac promoter of a plasmid pUCN18 (a plasmid obtained by modifying T at position 185 of pUC18 (manufactured by Takara-Bio Inc.) to A by means of PCR so as to destroy an NdeI site and further modifying GC at positions 471-472 to TG so as to newly introduce an NdeI site) to construct a recombinant vector pPPK.

(Reference Example 8) Preparation of recombinant organisms expressing polyphosphate kinase

With use of the recombinant vector pPPK constructed in Reference Example 7, an *E. coli* HB101 competent cell (manufactured by Takara-Bio Inc.) was transformed to obtain a recombinant organism *E. coli* HB101 (pPPK). In addition, with use of the pUCN18, an *E. coli* HB101 competent cell (manufactured by Takara-Bio Inc.) was transformed to obtain a recombinant organism *E. coli* HB101 (pUCN18).

(Reference Example 9) Expression of polyphosphate kinase gene in recombinant organisms

Two types of recombinant organisms (*E. coli* HB101 (pUCN18) and *E. coli* HB101 (pPPK) obtained in Reference Example 8 were each inoculated onto 5 ml of 2 × YT medium (tryptone: 1.6%, yeast extract: 1.0%, sodium chloride: 0.5%, pH: 7.0) containing 200 μg/ml of ampicillin, and were each subjected to shake culture at 37°C for 24 hours. Each culture solution thus obtained by the above culture was subjected to centrifugal separation to collect bacterial cells, and the bacterial cells were then suspended in 1ml of a 50 mM Tris-HCl buffer solution (pH: 8.0). Resultant suspensions were each disrupted by means of a UH-50 ultrasonic homogenizer (manufactured by SMT Co., Ltd.), and were then subjected to centrifugation so as to remove bacterial cell debris. Thus obtained were cell-free extracts. Polyphosphate kinase activity of each of these cell-free extracts was measured. Polyphosphate kinase activity was quantified through HPLC analysis of ATP produced by adding 5 mM sodium metaphosphate (manufactured by Wako Pure Chemical Industries, Ltd.), 10 mM ADP disodium salt (manufactured by Oriental Yeast Co., Ltd.), 70 mM magnesium sulfate (manufactured by Wako Pure Chemical Industries, Ltd.), and each of the cell-free extracts to a 50 mM Tris-HCl buffer solution (pH: 8.0) and then carrying out reaction at 30°C for 5 minutes. In this reaction condition, enzyme activity of producing 1 μmol of ATP for 1 minute was defined as 1U. ATP formation activities of the cell-free extracts of the respective recombinant organisms are shown below. For the *E. coli* HB101 (pUCN18), ATP formation activity was not more than 0.1 mU/mg. Meanwhile, for the *E. coli* HB101 (pPPK) which expressed polyphosphate kinase, ATP formation activity was 160 U/mg. As described above, the recombinant organisms obtained in Reference Example 8 were confirmed to have ATP formation activity and produce polyphosphate kinase.

(Reference Example 10) Preparation of polyphosphate kinase

The *E. coli* HB101 (pPPK) obtained in Reference Example 8 was inoculated onto 50 ml of 2YT medium (tryptone: 1.6%, yeast extract: 1.0%, NaCl: 0.5%, pH: 7.0) containing 200 μg/ml of ampicillin, and was subjected to shake culture at 37°C for 24 hours. Enzyme activity was measured by the method described in (Reference Example 9) was 110 U/mL. Subsequently, the bacterial cells were collected by centrifugation, suspended in 2.5 ml of a 50 mM Tris-HCl buffer solution (pH: 8.0), and sonicated to obtain an enzyme solution.

(Example 22) Production 2 of glutathione with use of modified glutathione synthetase

Oxidized γ-glutamyl cysteine was synthesized by the method described in <Example 1> of International Publication No. 2016/002884. To a reaction solution obtained after 22 hours of this reaction were added 0.19 g (2.53 mmol) of glycine, 2 g of the modified glutathione synthetase (V260A) prepared in Reference Example 6, and 2 g of polyphosphate kinase enzyme solution prepared in Reference Example 9. Then, reaction was started. At this time, pH was adjusted to 7.5 with 1.1 g of a 15 mass% aqueous sodium hydroxide solution. Analysis of a reaction solution obtained after 1 hour of reaction confirmed formation of oxidized glutathione. The yields were 33 mol% after 2 hours of reaction and 64 mol% after 8 hours of reaction, relative to starting L-cystine.

## Claims

**1.** A polypeptide exhibiting properties (a) and (b) below:

(a) being capable of carrying out a reaction of binding glycine to γ-glutamyl dipeptide; and
(b) having a higher thermal stability and/or a higher storage stability as compared with glutathione synthetase consisting of an amino acid sequence of SEQ ID NO: 1 shown in the Sequence Listing.

**2.** A polypeptide exhibiting properties (c) and (d) below:

(c) being capable of producing reduced glutathione (GSH) and/or oxidized glutathione (GSSG); and
(d) having a higher thermal stability and/or a higher storage stability as compared with glutathione synthetase consisting of an amino acid sequence of SEQ ID NO: 1 shown in the Sequence Listing.

**3.** A polypeptide according to claim 1, wherein the polypeptide is capable of: producing GSH and GSSG with use of γ-glutamyl cysteine and oxidized γ-glutamyl cysteine as substrates; producing GSH with use of γ-glutamyl cysteine as a substrate; or producing GSSG with use of oxidized γ-glutamyl cysteine as a substrate.

**4.** A polypeptide defined in any one of (A) to (C) below:

(A) a polypeptide according to any one of claims 1 to 3, wherein the polypeptide consists of an amino acid sequence which is obtained by substitution of one or more amino acids in the amino acid sequence of SEQ ID NO: 1 shown in the Sequence Listing, the one or more amino acids being selected from the group consisting of amino acids at respective positions 13, 17, 20, 23, 39, 70, 78, 101, 113, 125, 126, 136, 138, 149, 152, 154, 155, 197, 200, 215, 226, 227, 230, 239, 241, 246, 249, 254, 260, 262, 263, 270, 278, 299, 305, 307, and 310;
(B) a polypeptide according to any one of claims 1 to 3, wherein the polypeptide consists of an amino acid sequence which is obtained by substitution of one or more amino acids in the amino acid sequence of SEQ ID NO: 1 shown in the Sequence Listing, the one or more amino acids being selected from the group consisting of amino acids at respective positions 13, 17, 20, 23, 39, 70, 78, 101, 113, 125, 126, 136, 138, 149, 152, 154, 155, 197, 200, 215, 226, 227, 230, 239, 241, 246, 249, 254, 260, 262, 263, 270, 278, 299, 305, 307, and 310, and in which one or more amino acids at a position(s) other than said positions are substituted, added, inserted, or deleted; and
(C) a polypeptide according to any one of claims 1 to 3, wherein the polypeptide consists of an amino acid sequence which is obtained by substitution of one or more amino acids in the amino acid sequence of SEQ ID NO: 1 shown in the Sequence Listing, the one or more amino acids being selected from the group consisting of amino acids at respective positions 13, 17, 20, 23, 39, 70, 78, 101, 113, 125, 126, 136, 138, 149, 152, 154, 155, 197, 200, 215, 226, 227, 230, 239, 241, 246, 249, 254, 260, 262, 263, 270, 278, 299, 305, 307, and 310, and which has a sequence identity of 80% or more with respect to the amino acid sequence of SEQ ID NO: 1 shown in the Sequence Listing except for said positions.

**5.** A polypeptide defined in any one of (D) to (F) below:

(D) a polypeptide according to any one of claims 1 to 3, wherein the polypeptide consists of an amino acid sequence which is obtained by substitution of one or more amino acids in the amino acid sequence of SEQ ID NO: 1 shown in the Sequence Listing, the substitution of the one or more amino acids being selected from the group consisting of: substitution of an amino acid at position 13 to serine; substitution of an amino acid at position 17 to glutamic acid; substitution of an amino acid at position 20 to threonine; substitution of an amino acid at position 23 to leucine; substitution of an amino acid at position 39 to threonine; substitution of an amino acid at position 70 to serine; substitution of an amino acid at position 78 to leucine; substitution of an amino acid at position 101 to asparagine, glutamine, serine, or threonine; substitution of an amino acid at position 113 to histidine; substitution of an amino acid at position 125 to valine; substitution of an amino acid at position 126 to asparagine; substitution of an amino acid at position 136 to threonine; substitution of an amino acid at position 138 to alanine; substitution of an amino acid at position 149 to glutamine; substitution of an amino acid at position 152 to glutamine; substitution of an amino acid at position 154 to asparagine; substitution of an amino acid at position 155 to leucine; substitution of an amino acid at position 197 to glutamine; substitution of an amino acid at position 200 to serine; substitution of an amino acid at position 215 to asparagine acid; substitution of an amino acid at position 226 to arginine; substitution of an amino acid at position 227 to serine; substitution of an amino acid at position 230 to proline; substitution of an amino acid at position 239 to serine; substitution of an amino acid at position 241 to histidine; substitution of an amino acid at position 246 to arginine; substitution of an amino acid at position 249 to glutamic acid; substitution of an amino acid at position 254 to asparagine acid; substitution of an amino acid at position 260 to alanine, cystein, glycine, glutamine, or threonine; substitution of an amino acid at position 262 to cysteine; substitution of an amino acid at position 263 to arginine; substitution of an amino acid at position 270 to isoleucine; substitution of an amino acid at a position 278 to glycine or alanine; substitution of an amino acid at position 299 to alanine; substitution of an amino acid at position 305 to glycine; substitution of an amino acid at position 307 to valine; and substitution of an amino acid at position 310 to threonine; and
(E) a polypeptide according to any one of claims 1 to 3, wherein the polypeptide consists of an amino acid sequence which is obtained by substitution of one or more amino acids in the amino acid sequence of SEQ ID NO: 1 shown in the Sequence Listing, the substitution of the one or more amino acids being selected from the group consisting of: substitution of an amino acid at position 13 to serine; substitution of an amino acid at position 17 to glutamic acid; substitution of an amino acid at position 20 to threonine; substitution of an amino acid at position 23 to leucine; substitution of an amino acid at position 39 to threonine; substitution of an amino acid at position 70 to serine; substitution of an amino acid at position 78 to leucine; substitution of an amino acid at position 101 to asparagine, glutamine, serine, or threonine; substitution of an amino acid at position 113 to histidine; substitution of an amino acid at position 125 to valine; substitution of an amino acid at position 126 to asparagine; substitution of an amino acid at position 136 to threonine; substitution of an amino acid at position 138 to alanine; substitution of an amino acid at position 149 to glutamine; substitution of an amino acid at position 152 to glutamine; substitution of an amino acid at position 154 to asparagine; substitution of an amino acid at position 155 to leucine; substitution of an amino acid at position 197 to glutamine; substitution of an amino acid at position 200 to serine; substitution of an amino acid at position 215 to asparagine acid; substitution of an amino acid at position 226 to arginine; substitution of an amino acid at position 227 to serine; substitution of an amino acid at position 230 to proline; substitution of an amino acid at position 239 to serine; substitution of an amino acid at position 241 to histidine; substitution of an amino acid at position 246 to arginine; substitution of an amino acid at position 249 to glutamic acid; substitution of an amino acid at position 254 to asparagine acid; substitution of an amino acid at position 260 to alanine, cystein, glycine, glutamine, or threonine; substitution of an amino acid at position 262 to cysteine; substitution of an amino acid at position 263 to arginine; substitution of an amino acid at position 270 to isoleucine; substitution of an amino acid at a position 278 to glycine or alanine; substitution of an amino acid at position 299 to alanine; substitution of an amino acid at position 305 to glycine; substitution of an amino acid at position 307 to valine; and substitution of an amino acid at position 310 to threonine, and in which one or more amino acids at a position(s) other than said positions are substituted, added, inserted, or deleted; and

(F) a polypeptide according to any one of claims 1 to 3, wherein the polypeptide consists of an amino acid sequence which is obtained by substitution of one or more amino acids in the amino acid sequence of SEQ ID NO: 1 shown in the Sequence Listing, the substitution of the one or more amino acids being selected from the group consisting of: substitution of an amino acid at position 13 to serine; substitution of an amino acid at position 17 to glutamic acid; substitution of an amino acid at position 20 to threonine; substitution of an amino acid at position 23 to leucine; substitution of an amino acid at position 39 to threonine; substitution of an amino acid at position 70 to serine; substitution of an amino acid at position 78 to leucine; substitution of an amino acid at position 101 to asparagine, glutamine, serine, or threonine; substitution of an amino acid at position 113 to histidine; substitution of an amino acid at position 125 to valine; substitution of an amino acid at position 126 to asparagine; substitution of an amino acid at position 136 to threonine; substitution of an amino acid at position 138 to alanine; substitution of an amino acid at position 149 to glutamine; substitution of an amino acid at position 152 to glutamine; substitution of an amino acid at position 154 to asparagine; substitution of an amino acid at position 155 to leucine; substitution of an amino acid at position 197 to glutamine; substitution of an amino acid at position 200 to serine; substitution of an amino acid at position 215 to asparagine acid; substitution of an amino acid at position 226 to arginine; substitution of an amino acid at position 227 to serine; substitution of an amino acid at position 230 to proline; substitution of an amino acid at position 239 to serine; substitution of an amino acid at position 241 to histidine; substitution of an amino acid at position 246 to arginine; substitution of an amino acid at position 249 to glutamic acid; substitution of an amino acid at position 254 to asparagine acid; substitution of an amino acid at position 260 to alanine, cystein, glycine, glutamine, or threonine; substitution of an amino acid at position 262 to cysteine; substitution of an amino acid at position 263 to arginine; substitution of an amino acid at position 270 to isoleucine; substitution of an amino acid at a position 278 to glycine or alanine; substitution of an amino acid at position 299 to alanine; substitution of an amino acid at position 305 to glycine; substitution of an amino acid at position 307 to valine; and substitution of an amino acid at position 310 to threonine, and which has a sequence identity of 80% or more with respect to the amino acid sequence of SEQ ID NO: 1 shown in the Sequence Listing except for said positions.

**6.** A polypeptide defined in any one of (G) to (I) below:

(G) a polypeptide according to any one of claims 1 to 3, wherein the polypeptide consists of an amino acid sequence which is obtained by amino acid substitution in the amino acid sequence of SEQ ID NO: 1 shown in the Sequence Listing, the amino acid substitution being selected from the group consisting of:

(1) substitution of an amino acid at position 13 to serine;
(2) substitution of an amino acid at position 17 to glutamic acid, an amino acid at position 113 to histidine, and an amino acid at position 230 to proline;
(3) substitution of an amino acid at position 20 to threonine and an amino acid at position 215 to asparagine acid;
(4) substitution of an amino acid at position 20 to threonine and an amino acid at position 241 to histidine;
(5) substitution of an amino acid at position 23 to leucine and an amino acid at position 126 to asparagine;
(6) substitution of an amino acid at position 39 to threonine and an amino acid at position 260 to alanine;
(7) substitution of an amino acid at position 70 to serine and an amino acid at position 260 to alanine;
(8) substitution of an amino acid at position 78 to leucine and an amino acid at position 278 to alanine;
(9) substitution of an amino acid at position 101 to asparagine;
(10) substitution of an amino acid at position 101 to glutamine;
(11) substitution of an amino acid at position 101 to serine;
(12) substitution of an amino acid at position 101 to serine and an amino acid at position 260 to alanine;
(13) substitution of an amino acid at position 101 to threonine;

(14) substitution of an amino acid at position 125 to valine and an amino acid at position 249 to glutamic acid;
(15) substitution of an amino acid at position 125 to valine and an amino acid at position 152 to glutamine;
(16) substitution of an amino acid at position 136 to threonine;
(17) substitution of an amino acid at position 138 to alanine, an amino acid at position 149 to glutamine, an amino acid at position 241 to histidine, and an amino acid at position 263 to glutamine;
(18) substitution of an amino acid at position 154 to asparagine and an amino acid at position 246 to arginine;
(19) substitution of an amino acid at position 155 to leucine and an amino acid at position 239 to serine;
(20) substitution of an amino acid at position 197 to glutamine;
(21) substitution of an amino acid at position 200 to serine and an amino acid at position 260 to alanine;
(22) substitution of an amino acid at position 226 to arginine and an amino acid at position 260 to alanine;
(23) substitution of an amino acid at position 227 to serine and an amino acid at position 260 to alanine;
(24) substitution of an amino acid at position 254 to asparagine acid and an amino acid at position 260 to alanine;
(25) substitution of an amino acid at position 260 to alanine;
(26) substitution of an amino acid at position 260 to alanine, an amino acid at position 278 to glycine, and an amino acid at position 307 to valine;
(27) substitution of an amino acid at position 260 to alanine and an amino acid at position 299 to alanine;
(28) substitution of an amino acid at position 260 to alanine and an amino acid at position 305 to glycine;
(29) substitution of an amino acid at position 260 to alanine and an amino acid at position 310 to threonine;
(30) substitution of an amino acid at position 260 to cysteine;
(31) substitution of an amino acid at position 260 to glycine;
(32) substitution of an amino acid at position 260 to glutamine;
(33) substitution of an amino acid at position 260 to threonine;
(34) substitution of an amino acid at position 262 to cysteine; and
(35) substitution of an amino acid at position 270 to isoleucine;

(H) a polypeptide according to any one of claims 1 to 3, wherein the polypeptide consists of an amino acid sequence which is obtained by amino acid substitution in the amino acid sequence of SEQ ID NO: 1 shown in the Sequence Listing, the amino acid substitution being selected from the group consisting of:

(1) substitution of an amino acid at position 13 to serine;
(2) substitution of an amino acid at position 17 to glutamic acid, an amino acid at position 113 to histidine, and an amino acid at position 230 to proline;
(3) substitution of an amino acid at position 20 to threonine and an amino acid at position 215 to asparagine acid;
(4) substitution of an amino acid at position 20 to threonine and an amino acid at position 241 to histidine;
(5) substitution of an amino acid at position 23 to leucine and an amino acid at position 126 to asparagine;
(6) substitution of an amino acid at position 39 to threonine and an amino acid at position 260 to alanine;
(7) substitution of an amino acid at position 70 to serine and an amino acid at position 260 to alanine;
(8) substitution of an amino acid at position 78 to leucine and an amino acid at position 278 to alanine;
(9) substitution of an amino acid at position 101 to asparagine;
(10) substitution of an amino acid at position 101 to glutamine;
(11) substitution of an amino acid at position 101 to serine;
(12) substitution of an amino acid at position 101 to serine and an amino acid at position 260 to alanine;
(13) substitution of an amino acid at position 101 to threonine;
(14) substitution of an amino acid at position 125 to valine and an amino acid at position 249 to glutamic acid;
(15) substitution of an amino acid at position 125 to valine and an amino acid at position 152 to glutamine;
(16) substitution of an amino acid at position 136 to threonine;
(17) substitution of an amino acid at position 138 to alanine, an amino acid at position 149 to glutamine, an amino acid at position 241 to histidine, and an amino acid at position 263 to glutamine;
(18) substitution of an amino acid at position 154 to asparagine and an amino acid at position 246 to arginine;
(19) substitution of an amino acid at position 155 to leucine and an amino acid at position 239 to serine;
(20) substitution of an amino acid at position 197 to glutamine;
(21) substitution of an amino acid at position 200 to serine and an amino acid at position 260 to alanine;
(22) substitution of an amino acid at position 226 to arginine and an amino acid at position 260 to alanine;
(23) substitution of an amino acid at position 227 to serine and an amino acid at position 260 to alanine;
(24) substitution of an amino acid at position 254 to asparagine acid and an amino acid at position 260 to alanine;

(25) substitution of an amino acid at position 260 to alanine;
(26) substitution of an amino acid at position 260 to alanine, an amino acid at position 278 to glycine, and an amino acid at position 307 to valine;
(27) substitution of an amino acid at position 260 to alanine and an amino acid at position 299 to alanine;
(28) substitution of an amino acid at position 260 to alanine and an amino acid at position 305 to glycine;
(29) substitution of an amino acid at position 260 to alanine and an amino acid at position 310 to threonine;
(30) substitution of an amino acid at position 260 to cysteine;
(31) substitution of an amino acid at position 260 to glycine;
(32) substitution of an amino acid at position 260 to glutamine;
(33) substitution of an amino acid at position 260 to threonine;
(34) substitution of an amino acid at position 262 to cysteine; and
(35) substitution of an amino acid at position 270 to isoleucine, and in which one or more amino acids at a position(s) other than said positions are substituted, added, inserted, or deleted; and

(I) a polypeptide according to any one of claims 1 to 3, wherein the polypeptide consists of an amino acid sequence which is obtained by amino acid substitution in the amino acid sequence of SEQ ID NO: 1 shown in the Sequence Listing, the amino acid substitution being selected from the group consisting of:

(1) substitution of an amino acid at position 13 to serine;
(2) substitution of an amino acid at position 17 to glutamic acid, an amino acid at position 113 to histidine, and an amino acid at position 230 to proline;
(3) substitution of an amino acid at position 20 to threonine and an amino acid at position 215 to asparagine acid;
(4) substitution of an amino acid at position 20 to threonine and an amino acid at position 241 to histidine;
(5) substitution of an amino acid at position 23 to leucine and an amino acid at position 126 to asparagine;
(6) substitution of an amino acid at position 39 to threonine and an amino acid at position 260 to alanine;
(7) substitution of an amino acid at position 70 to serine and an amino acid at position 260 to alanine;
(8) substitution of an amino acid at position 78 to leucine and an amino acid at position 278 to alanine;
(9) substitution of an amino acid at position 101 to asparagine;
(10) substitution of an amino acid at position 101 to glutamine;
(11) substitution of an amino acid at position 101 to serine;
(12) substitution of an amino acid at position 101 to serine and an amino acid at position 260 to alanine;
(13) substitution of an amino acid at position 101 to threonine;
(14) substitution of an amino acid at position 125 to valine and an amino acid at position 249 to glutamic acid;
(15) substitution of an amino acid at position 125 to valine and an amino acid at position 152 to glutamine;
(16) substitution of an amino acid at position 136 to threonine;
(17) substitution of an amino acid at position 138 to alanine, an amino acid at position 149 to glutamine, an amino acid at position 241 to histidine, and an amino acid at position 263 to glutamine;
(18) substitution of an amino acid at position 154 to asparagine and an amino acid at position 246 to arginine;
(19) substitution of an amino acid at position 155 to leucine and an amino acid at position 239 to serine;
(20) substitution of an amino acid at position 197 to glutamine;
(21) substitution of an amino acid at position 200 to serine and an amino acid at position 260 to alanine;
(22) substitution of an amino acid at position 226 to arginine and an amino acid at position 260 to alanine;
(23) substitution of an amino acid at position 227 to serine and an amino acid at position 260 to alanine;
(24) substitution of an amino acid at position 254 to asparagine acid and an amino acid at position 260 to alanine;
(25) substitution of an amino acid at position 260 to alanine;
(26) substitution of an amino acid at position 260 to alanine, an amino acid at position 278 to glycine, and an amino acid at position 307 to valine;
(27) substitution of an amino acid at position 260 to alanine and an amino acid at position 299 to alanine;
(28) substitution of an amino acid at position 260 to alanine and an amino acid at position 305 to glycine;
(29) substitution of an amino acid at position 260 to alanine and an amino acid at position 310 to threonine;
(30) substitution of an amino acid at position 260 to cysteine;
(31) substitution of an amino acid at position 260 to glycine;
(32) substitution of an amino acid at position 260 to glutamine;
(33) substitution of an amino acid at position 260 to threonine;
(34) substitution of an amino acid at position 262 to cysteine; and
(35) substitution of an amino acid at position 270 to isoleucine, and which has a sequence identity of 80% or more with respect to the amino acid sequence of SEQ ID NO: 1 shown in the Sequence Listing except for said positions.

**7.** A polynucleotide encoding a polypeptide according to any one of claims 1 to 6.

**8.** A method for producing γ-Glu-X-Gly where X represents an amino acid, the method comprising causing a transformant expressing a polypeptide according to any one of claims 1 to 6 or a polynucleotide according to claim 7 and/or a treated material of the transformant to act on γ-glutamyl dipeptide.

**9.** A method for producing GSSG, comprising causing a transformant expressing a polypeptide according to any one of claims 1 to 6 or a polynucleotide according to claim 7 and/or a treated material of the transformant to act on oxidized γ-glutamyl cysteine.

**10.** A method for producing GSH, comprising causing a transformant expressing a polypeptide according to any one of claims 1 to 6 or a polynucleotide according to claim 7 and/or a treated material of the transformant to act on γ-glutamyl cysteine.

**11.** The method according to claim 8, wherein a reaction is carried out in the coexistence of an ATP regenerating system.

**12.** The method according to claim 11, wherein polyphosphate kinase is used as the ATP regenerating system.

**13.** The method according to claim 9, wherein a reaction is carried out in the coexistence of an ATP regenerating system.

**14.** The method according to claim 13, wherein polyphosphate kinase is used as the ATP regenerating system.

**15.** The method according to claim 10, wherein a reaction is carried out in the coexistence of an ATP regenerating system.

**16.** The method according to claim 15, wherein polyphosphate kinase is used as the ATP regenerating system.

## INTERNATIONAL SEARCH REPORT

International application No.
PCT/JP2017/039473

A. CLASSIFICATION OF SUBJECT MATTER
Int.Cl. C12N15/09(2006.01)i, C12N9/02(2006.01)i, C12P21/02(2006.01)n

According to International Patent Classification (IPC) or to both national classification and IPC

B. FIELDS SEARCHED
Minimum documentation searched (classification system followed by classification symbols)
Int.Cl. C12N15/09, C12N9/02, C12P21/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580(JDreamIII), CAplus/WPIDS/MEDLINE/EMBASE/BIOSIS(STN), UniProt/GeneSeq

C. DOCUMENTS CONSIDERED TO BE RELEVANT

| Category* | Citation of document, with indication, where appropriate, of the relevant passages | Relevant to claim No. |
|---|---|---|
| Y | Glutathione synthetase [Thiobacillus denitrificans ATCC 25259], GenBank[online], accession no. AAZ98362, [retrieved on 10 January 2018], 28 January 2014, retrieved from the Internet: http://www.ncbi.nlm.nih.gov/protein/74057922/ | 1-16 |
| Y | JP 2015-084676 A (TOYOBO CO., LTD.) 07 May 2015, paragraph [0006], examples 1-3 (Family: none) | 1-16 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 January 2018 | 30 January 2018 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2017/039473

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2014-209898 A (TOSOH CORPORATION) 13 November 2014, examples 1-15<br>& WO 2014/104094 A1 | 1-16 |
| Y | WO 2015/099112 A1 (KIKKOMAN CORPORATION) 02 July 2015, paragraph [0022], examples 1-4<br>& US 2016/0319246 A1, paragraph [0099], examples 1-4 | 1-16 |
| A | WO 2013/054447 A1 (AJINOMOTO CO., INC.) 18 April 2013, paragraph [0018]<br>(Family: none) | 1-16 |
| A | JP 2012-085637 A (AJINOMOTO CO., INC.) 10 May 2012, paragraph [0037]<br>(Family: none) | 1-16 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 60027396 A
- JP 60027397 A
- WO 2016002884 A
- WO 9403613 A
- WO 2006080313 A


### Non-patent literature cited in the description


- *Appl. Microbiol. Biotechnol.,* 2004, vol. 66, 233
- *Appl. Environ. Microbiol.,* 1982, vol. 44, 1444
- **JOSEPH SAMBROOK.** Molecular Cloning. Cold Spring Harbor Laboratory Press, 1989
- *Pure & Appl. Chem.,* 1991, vol. 63 (10), 1527-1540
- **LEUNG et al.** *Technique,* 1989, vol. 1, 11-15
- **FREDERICK M. AUSUBEL.** Current Protocols in Molecular Biology. Greene Publishing Associates and Wiley-Interscience, 1989
- **OLFERT LANDT et al.** *Gene,* 1990, vol. 96, 125-128
- **SMITH et al.** Genetic Engineering. J. Plenum Press, vol. 3, 1
- **VLASUK et al.** Experimental Manipulation of Gene Expression. Academic Press
- **HOS. N. HUNT et al.** *Gene,* 1989, vol. 77, 51
- **ANDO TADAHIKO.** Biseibutsugaku Kiso Koza. KY-ORITSU SHUPPAN, 1987, vol. 8
- *Nature,* 1985, vol. 315, 592-594

SEQUENCE LISTING

<110> KANEKA CORPORATION

<120> Modified enzyme and use of same

<130> B160880

<150> JP 2016-214073
<151> 2016-11-01

<160> 20

<170> PatentIn version 3.5

<210> 1
<211> 314
<212> PRT
<213> Thiobacillus denitrificans

<400> 1

```
Met Lys Leu Leu Phe Val Val Asp Pro Leu Ala Ser Leu Lys Pro Tyr
1               5                   10                  15

Lys Asp Ser Ser Val Ala Met Met Arg Ala Ala Cys Ala Arg Gly His
            20                  25                  30

Ala Val Phe Ala Ala Glu Ala Arg Ala Leu Leu Val Arg Asp Gly Val
        35                  40                  45

Ala Arg Ser Arg Ala Asp Ala Val Glu Thr Arg Gly Asp Asp Asp Trp
    50                  55                  60

Tyr Arg Val Thr Glu Thr Arg Glu Phe Ala Leu Thr Asp Phe Asp Ala
65                  70                  75                  80

Val Val Met Arg Ala Asp Pro Pro Val Asp Val Asp Tyr Leu Leu Ala
                85                  90                  95

Thr His Leu Leu Gly Val Ala Glu Thr Asn Gly Ala Arg Val Leu Asn
            100                 105                 110

Arg Pro Arg Ala Leu Arg Asp Phe Asn Glu Lys Leu Ala Ile Leu Glu
        115                 120                 125

Phe Pro Gln Phe Val Ala Pro Thr Leu Val Ser Ala Asp Ala Thr Glu
    130                 135                 140

Ile Ala His Phe Leu Ala Ala His Ala Asp Ile Ile Val Lys Pro Leu
145                 150                 155                 160

Thr Glu Met Gly Gly Ser Gly Val Phe Arg Leu Gly Val Ser Asp Pro
```

165 170 175

Asn Arg Asn Ala Ile Leu Glu Thr Leu Thr Arg Arg Gly Ser Arg Pro
180 185 190

Ile Met Ala Gln Arg Tyr Leu Pro Ala Ile Ser Glu Gly Asp Lys Arg
195 200 205

Ile Leu Leu Ile Asp Gly Glu Val Val Pro Trp Ala Leu Ala Arg Ile
210 215 220

Pro Leu Thr Gly Glu Thr Arg Gly Asn Leu Ala Ala Gly Gly Thr Ala
225 230 235 240

Arg Ala Gln Pro Leu Ser Glu Arg Asp Arg Glu Ile Ala Glu Thr Ile
245 250 255

Ala Pro Trp Val Arg Ser Gln Gly Ile Phe Leu Ala Gly Leu Asp Val
260 265 270

Ile Gly Asp Cys Leu Thr Glu Ile Asn Val Thr Ser Pro Thr Gly Phe
275 280 285

Gln Glu Ile Thr Ala Gln Ser Gly His Asp Val Ala Asp Gln Phe Ile
290 295 300

Ala Ala Ile Glu Arg Ala Thr Arg Pro Glu
305 310

<210> 2
<211> 945
<212> DNA
<213> Thiobacillus denitrificans

<400> 2
atgaagctgc tgttcgtcgt cgatccgctg gcgagcctca agccgtacaa ggacagttcg 60

gtcgcgatga tgcgcgcggc ctgtgcgcgc ggccacgccg tgttcgcggc cgaagcacgc 120

gcgctgctgg tgcgcgacgg agtcgcgcgg tcgcgcgccg acgccgtcga gacgcgcggc 180

gacgacgatt ggtatcgcgt gaccgaaacg cgcgaattcg cgctcacgga tttcgatgcc 240

gttgtgatgc gcgccgaccc gccggtcgac gtcgactacc tgctcgcgac ccacctgctc 300

ggcgtcgccg agaccaacgg cgcgcgcgtg ctgaaccggc cgcgcgcgct gcgcgacttc 360

aacgaaaaac tcgccatcct cgagtttccg caatttgtcg ccccgacgct ggtttcggcc 420

gacgcgacag aaatcgccca cttcctcgcc gcccacgccg acatcatcgt caagccgctc 480

accgagatgg gcggcagcgg cgtcttccgc ctcggcgttt ccgacccgaa ccgcaacgcc 540

atcctcgaaa cgctcacccg acgcggcagc cggccgatca tggcgcagcg ttatctgccg 600

gcgatcagcg aaggcgacaa gcgcatcctg ctgatcgacg gcgaggtggt gccgtgggcc 660

ctcgcgcgga ttccgctgac gggcgagacg cgcggcaatc tcgccgcggg cggcacggcg 720

cgtgcccagc cgctttcgga acgcgaccgc gagatcgccg agacgatcgc gccctgggtg 780

cgctcgcaag gcatcttcct cgccggcctc gacgtgatcg gcgactgcct caccgaaatc 840

aacgtcacga gcccgaccgg ctttcaggaa atcacggcgc agagcggcca cgacgtcgcg 900

gaccagttca tcgccgcgat cgagcgtgcc acgcgtccgg aataa 945

<210> 3
<211> 948
<212> DNA
<213> Artificial Sequence

<220>
<223> Optimized sequence for glutahione synthetase of Thiobacillus denitrificans

<400> 3
atgaaactgc tgttcgtcgt tgatcccctg gccagcttga aaccgtacaa ggatagctcc 60

gttgccatga tgcgcgcagc gtgtgctcgt ggtcatgccg tgttcgcagc agaagcgcgc 120

gcactgctgg ttcgtgatgg ggtggctcgt tctcgtgcag atgctgtcga aacgcgtggc 180

gacgatgact ggtatcgcgt taccgaaacg cgtgaatttg ccttaaccga ctttgatgca 240

gtggtgatgc gcgcagatcc gcccgttgac gtggattacc ttctcgcgac gcacctgtta 300

ggcgtagccg aaaccaacgg tgcacgtgtc ctgaatcgcc cgcgtgcctt gcgcgatttc 360

aacgagaaac tggccattct ggaatttccg cagtttgtcg cacctaccct ggtaagtgcg 420

gacgcaaccg aaattgccca ctttctggct gctcatgcgg atatcatcgt caaaccgctg 480

actgagatgg gtggctccgg tgtgtttcgc ctgggagtta gcgatccgaa tcggaacgcg 540

attctggaga cattaacccg tcgtggctct cgcccaatca tggctcagcg gtatttgcca 600

gcgatctcag agggcgacaa acgcatcctg ctgatcgacg gcgaagtagt gccatgggcc 660

ttggcgcgca ttccgctgac cggtgaaact cgcgggaatc ttgcggctgg tggtacagcg 720

cgcgcgcaac cgctcagtga acgggatcgc gaaatcgccg aaacgattgc cccttgggta 780

cgcagccagg gcattttcct tgcgggctta gacgtgattg gggattgcct caccgagatt 840

aacgtgacat cgcctactgg atttcaggag attaccgccc aatcgggcca tgatgttgcg 900

gaccagttca ttgcggcgat cgaacgcgcg acgcgtccgg aatgataa 948

<210> 4
<211> 28
<212> DNA
<213> Artificial Sequence

<220>
<223> Primer for error-prone PCR

<400> 4
gggtttcata tgaaactgct gttcgtcg 28

<210> 5
<211> 26
<212> DNA
<213> Artificial Sequence

<220>
<223> Primer for error-prone PCR

<400> 5
ccggaattct tatcattccg gacgcg 26

<210> 6
<211> 33
<212> DNA
<213> Artificial Sequence

<220>
<223> Primer for PCR to amplify a mutant gene

<400> 6
acgattgccc cttggtgtcg cagccagggc att 33

<210> 7
<211> 33
<212> DNA
<213> Artificial Sequence

<220>
<223> Primer for PCR to amplify a mutant gene

<400> 7
aatgccctgg ctgcgacacc aaggggcaat cgt 33

<210> 8
<211> 33
<212> DNA
<213> Artificial Sequence

<220>
<223> Primer for PCR to amplify a mutant gene

<400> 8
acgattgccc cttggggtcg cagccagggc att 33

<210> 9
<211> 33
<212> DNA
<213> Artificial Sequence

<220>
<223> Primer for PCR to amplify a mutant gene

<400> 9
aatgccctgg ctgcgacccc aaggggcaat cgt 33

<210> 10
<211> 33
<212> DNA
<213> Artificial Sequence

<220>
<223> Primer for PCR to amplify a mutant gene

<400> 10
acgattgccc cttggcagcg cagccagggc att 33

<210> 11
<211> 33
<212> DNA
<213> Artificial Sequence

<220>
<223> Primer for PCR to amplify a mutant gene

<400> 11
aatgccctgg ctgcgctgcc aaggggcaat cgt 33

<210> 12
<211> 33
<212> DNA
<213> Artificial Sequence

<220>
<223> Primer for PCR to amplify a mutant gene

<400> 12
acgattgccc cttggacacg cagccagggc att 33

<210> 13
<211> 33
<212> DNA
<213> Artificial Sequence

<220>
<223> Primer for PCR to amplify a mutant gene

<400> 13
aatgccctgg ctgcgtgtcc aaggggcaat cgt 33

<210> 14
<211> 33
<212> DNA
<213> Artificial Sequence

<220>
<223> Primer for PCR to amplify a mutant gene

<400> 14
gcgacgcacc tgttaaacgt agccgaaacc aac 33

<210> 15
<211> 33
<212> DNA
<213> Artificial Sequence

<220>
<223> Primer for PCR to amplify a mutant gene

<400> 15
gttggtttcg gctaccttta acaggtgcgt cgc 33

<210> 16
<211> 33
<212> DNA
<213> Artificial Sequence

<220>
<223> Primer for PCR to amplify a mutant gene

<400> 16
gcgacgcacc tgttacaggt agccgaaacc aac 33

<210> 17
<211> 33
<212> DNA
<213> Artificial Sequence

<220>
<223> Primer for PCR to amplify a mutant gene

<400> 17
gttggtttcg gctacctgta acaggtgcgt cgc 33

<210> 18
<211> 33
<212> DNA
<213> Artificial Sequence

<220>
<223> Primer for PCR to amplify a mutant gene

<400> 18
gcgacgcacc tgttaaccgt agccgaaacc aac 33

<210> 19
<211> 33
<212> DNA
<213> Artificial Sequence

<220>
<223> Primer for PCR to amplify a mutant gene

<400> 19
gttggtttcg gctacggtta acaggtgcgt cgc 33

<210> 20
<211> 834

<212> DNA
<213> Artificial Sequence

<220>
<223> Optimized sequence of mutant polyphosphate kinase

<400> 20
atgaactatc cgtaccatac gcgtatgcgt cgcaatgaat atgaaaaagc caaacacgac 60
ctgcaaattg aactgctgaa agttcagagc tgggtcaaag aaaccggcca gcgcgtggtt 120
gtcctgtttg aaggccgtga tgcggccggt aaaggcggta cgattaaacg cttcatggaa 180
catctgaacc cgcgtggcgc ccgtattgtt gcactggaaa aaccgagttc ccaggaacaa 240
ggccagtggt attttcaacg ttacatccag cacctgccga ccgcgggcga aatggtgttt 300
ttcgatcgtt cttggtataa ccgcgccggc gtggaacgtg ttatgggttt ttgcagtccg 360
ctgcaatacc tggaatttat gcgccaggcg ccggaactgg aacgtatgct gaccaacagc 420
ggtattctgc tgtttaaata ttggttctct gttagtcgcg aagaacagct gcgtcgcttt 480
atcagccgtc gcgatgaccc gctgaaacat tggaaactgt ccccgattga tatcaaatca 540
ctggacaaat gggatgacta caccgcagct aaacaggcaa tgtttttcca caccgatacg 600
gcagacgctc cgtggacggt gattaaatcc gatgacaaaa aacgtgcgcg cctgaattgt 660
atccgccatt tcctgcactc actggattac ccggataaag accgtcgcat tgctcatgaa 720
ccggatccgc tgctggttgg cccggcaagc cgtgttatcg aagaagatga aaaagtttac 780
gcagaagcag cagcagcacc gggtcacgcc aacctggaca ttccggcatg ataa 834